# EUROPEAN PATENT SPECIFICATION

(11) **EP 4 272 779 B1**
(45) Date of publication and mention of the grant of the patent: **13.05.2026**
(21) Application number: 21914571.1
(22) Date of filing: 29.12.2021
(51) Int. Cl.: A61M 5/142, A61B 18/12

(54) **RADIO FREQUENCY OPERATION DATA APPARATUS, AND SYRINGE PUMP**
VORRICHTUNG UND SPRITZENPUMPE ZUR STEUERUNG VON HOCHFREQUENZBETRIEBSDATEN
DISPOSITIF ET POMPE À SERINGUE POUR LE CONTRÔLE DES DONNÉES DE FONCTIONNEMENT À HAUTE FRÉQUENCE

(30) Priority: 31.12.2020 CN 202011642244
(43) Date of publication of application: 08.11.2023
(73) Proprietor: Hangzhou Broncus Medical Co., Ltd., Hangzhou, Zhejiang 310051 (CN)
(72) Inventor: XU, Hong, Hangzhou, Zhejiang 310051 (CN); CUI, Changjie, Hangzhou, Zhejiang 310051 (CN)
(74) Representative: Petraz, Gilberto Luigi
(86) International application number: PCT/CN2021/142750
(87) International publication number: WO 2022/143839

(56) References cited:
- CN-A- 1 596 085
- CN-A- 110 074 857
- CN-A- 110 897 710
- CN-A- 110 897 710
- CN-A- 112 641 501
- CN-A- 112 716 594
- CN-A- 112 791 262
- CN-U- 205 215 353
- US-A1- 2008 287 944
- US-A1- 2013 030 426
- US-A1- 2020 275 971
- US-B1- 6 402 742

## Description

### TECHNICAL FIELD

Embodiments of the present disclosure relate to the field of communication technologies, and in particular, to a radio frequency operation data control method, apparatus, and syringe pump.

### DESCRIPTION OF THE PRIOR ART

During the radio frequency operation, the physical characteristic parameters of the radio frequency operation object may change with the radio frequency operation. If they are not controlled in time, damage to the radio frequency operation object, failure of the radio frequency host and other equipment, abnormal radio frequency operation, and even harm to the radio frequency operator may occur. Chinese Patent Application Publication No. CN110897710A provides a control method and a control system of a pulmonary nerve ablation system, and a computer medium; U.S. Patent Application Publication No. US2020/275971A1 provides a method of body tissue ablation; U.S. Patent Application Publication No. US2008/287944A1 provides a method and apparatus for carrying our thermal ablation of target tissue; and U.S. Patent Application Publication No. US2013/030426A1 provides a catheter adapted for ablation.

In the prior arts, the operator usually controls the physical characteristic parameters of the radio frequency operation object by experience, which cannot form an effective protection for the radio frequency operation object, and there are often misjudgments and delays in operation, thereby reducing the effect and safety of radio frequency operation.

### SUMMARY OF THE DISCLOSURE

Methods of surgery or therapy hereafter do not form part of the claimed invention, which is defined by the independent claims. The embodiments of the present disclosure aim to provide a radio frequency operation data control method, apparatus, and syringe pump, which can reduce operational delays and errors caused by human judgment, and can also improve the timeliness, accuracy and pertinence of liquid perfusion during the radio frequency operation and improve the effectiveness and safety of the radio frequency operation.

In one aspect, the embodiments of the present disclosure provide an electronic apparatus according to claim 1.

In a further aspect, the embodiments of the present disclosure provide a syringe pump according to claim 11.

In the embodiments of the present disclosure, when the radio frequency operation task is triggered, the syringe pump is controlled to randomly open one perfusion channel, and perform the perfusion operation through the opened perfusion channel at the preset initial flow rate, and the control mode for the radio frequency operation data is determined. In case of a dual control mode, according to the temperatures of multiple different sites of the radio frequency operation object acquired in real time by the plurality of temperature acquisition devices, the syringe pump is controlled to open or close part or all of the perfusion channels and / or to adjust the flow rates of part or all of the perfusion channels; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature and impedance changes of multiple different sites of the radio frequency operation object. Since the perfusion volume of the syringe pump is adjusted more purposefully and directionally according to the real-time changes of the temperature and impedance values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

### BRIEF DESCRIPTION OF DRAWINGS

In order to more clearly illustrate the technical solutions of the embodiments of the present disclosure or the prior art, the drawings that need to be referred in the description of the embodiments or the prior art will be briefly introduced in the following. Obviously, the drawings in the following only show some embodiments of the present disclosure. For those skilled in the art, other drawings can also be obtained according to these drawings without any creative effort.
FIG. 1 is a schematic diagram of an application scenario of a radio frequency operation data control method according to an embodiment of the present disclosure;
FIG. 2 is a schematic diagram of the internal structure of the syringe pump according to an embodiment of the present disclosure;
FIG. 3 is a schematic diagram of the internal structure of the syringe arrangement of the syringe pump in FIG. 2;
FIG. 4 is a flowchart of a radio frequency operation data control method according to an embodiment of the present disclosure;
FIG. 5 is a flowchart of a radio frequency operation data control method according to another embodiment of the present disclosure;
FIG. 6 is a flowchart of a radio frequency operation data control method according to a further embodiment of the present disclosure;
FIG. 7 is a flowchart of a radio frequency operation data control method according to a still further embodiment of the present disclosure;
FIG. 8 is a schematic view of the layout of multiple perfusion channels and a plurality of temperature acquisition devices in the radio frequency operation data control method according to the embodiment of the present disclosure;
FIG. 9 is a schematic diagram of a control apparatus for multi-channel perfusion of a syringe pump according to an embodiment of the present disclosure;
FIG. 10 is a schematic structural diagram of hardware of an electronic apparatus according to an embodiment of the present disclosure.

### DESCRIPTION OF EMBODIMENTS

In order to make the purpose, technical solutions and advantages of the embodiments of the present disclosure apparent, the technical solutions of the embodiments of the present disclosure will be described clearly and completely in combination with the drawings accompanying the embodiments of the present disclosure. Obviously, the drawings described only show some embodiments of the present disclosure, but not all embodiments. Based on the embodiments of the present disclosure, all other embodiments obtained by those skilled in the art without creative efforts fall within the protection scope of the present disclosure.

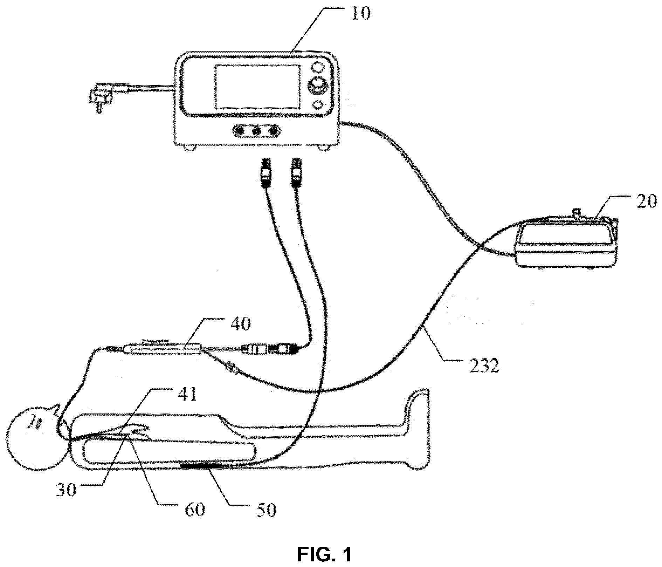

Referring to FIG. 1, a schematic diagram of an application scenario of a radio frequency operation data control method according to an embodiment of the present disclosure is shown. The radio frequency operation data control method can be implemented by the radio frequency host 10 or the syringe pump 20 in FIG. 1. Optionally, the radio frequency operation data control method can be implemented by other computer devices other than the radio frequency host 10 or the syringe pump 20, such as: server, desktop computer, notebook computer, laptop computer, tablet computer, personal computer and smart phone and the like.

As shown in FIG. 1, the radio frequency operating system includes a radio frequency host 10, a syringe pump 20 with multiple perfusion channels, a plurality of temperature acquisition devices 30, a radio frequency operation catheter 40, a neutral electrode 50 and an impedance acquisition device 60. The plurality of temperature acquisition devices 30 and the impedance acquisition device 60 may be arranged at the top of the radio frequency operation catheter 40, or be arranged at the top of the extension tube 232 of the syringe pump. The plurality of temperature acquisition devices 30 are respectively configured to acquire the temperatures of different positions of the operation site. The impedance acquisition device 60 is configured to acquire the impedance of the operation site of the radio frequency object.

Taking the radio frequency host 10 as the execution body of the radio frequency operation data control method according to the embodiment of the present disclosure as an example, specifically, firstly, the top of the radio frequency operation catheter 40 for generating and outputting radio frequency energy and the top of the extension tube 232 of the syringe pump 20 are inserted into the body of the radio frequency operation object, and reaches the operation site. The neutral electrode 50 is then brought into contact with the surface of the radio frequency operation object. The radio frequency current flows through the radio frequency operation catheter 40, the radio frequency operation object and the neutral electrode 50, thereby forming a loop. When the radio frequency operation task is triggered, the radio frequency host 10 controls the radio frequency operation catheter 40 to output radio frequency energy to the operation site by discharging, so as to perform radio frequency operation on the operation site.

At the same time, the radio frequency host 10 controls the syringe pump 20 to open at least one perfusion channel, so as to perform perfusion operation through the opened perfusion channel according to a preset initial flow rate, so as to perfuse liquid into the operation site. Then, when the control mode for the radio frequency operation data is a dual control mode, the temperatures of different positions of the operation site are acquired in real time through the plurality of temperature acquisition devices 30, and the syringe pump 20 is controlled according to the real-time changes of the temperature values of multiple sites to open or close part or all of the perfusion channels, and / or to adjust the flow rates of part or all of the perfusion channels.

Further, when the flow rate of the syringe pump 20 reaches the preset temperature-control limit, while the temperature values of the multiple sites exceed the temperature protection range, the output power of the radio frequency is controlled to make the temperature values of the multiple sites fall within the temperature protection range. At the same time, the impedance values of the multiple sites are detected, and when the impedance values of the multiple sites exceed the preset impedance protection range, the syringe pump 20 is controlled to adjust the flow rates of part or all of the perfusion channels, to make the impedance values of the multiple sites fall within the impedance protection range.

The radio frequency operation object may be any object requiring radio frequency operation. For example, when the radio frequency host 10 is a radio frequency ablation apparatus, the radio frequency operation object may be an animal body the abnormal tissues in which need to be ablated.

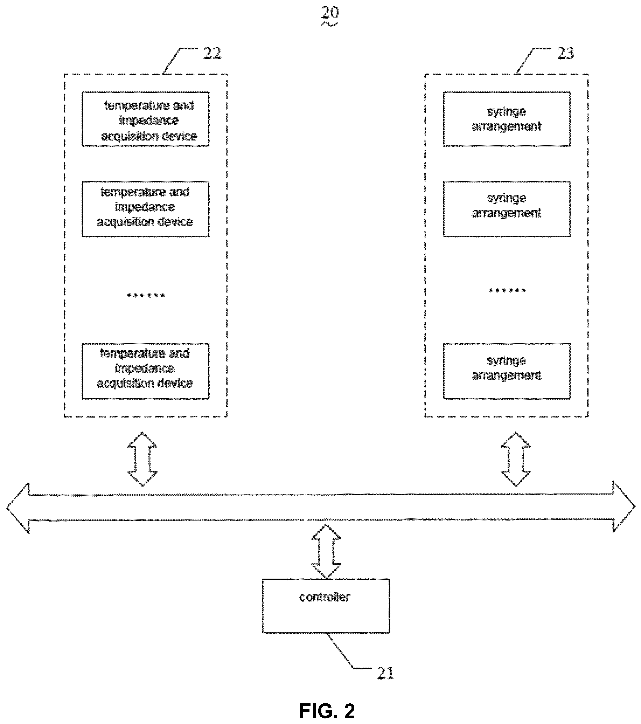

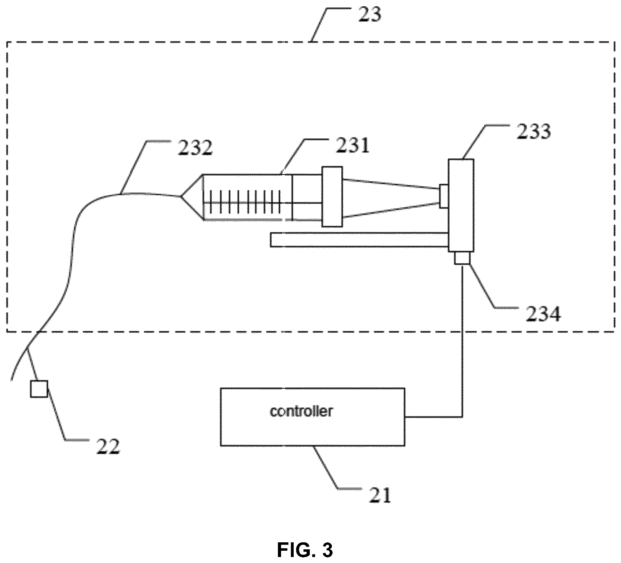

Referring to FIG. 2 and FIG. 3, FIG. 2 is a schematic diagram of the internal structure of the syringe pump according to an embodiment of the present disclosure, and FIG. 3 is a schematic diagram of the internal structure of the syringe arrangement 23 in FIG. 2. For illustration, FIG. 2 and FIG. 3 only show some structures related to this embodiment, and in practice, more or less structures than those shown in FIG. 3 may be used. As shown in FIGS. 2 and 3, the syringe pump 20 includes a controller 21, multiple temperature and impedance acquisition devices 22 and multiple syringe arrangements 23.

The temperature and impedance acquisition devices 22 include a temperature acquisition circuit and an impedance acquisition circuit, which are respectively used to acquire the temperature and the impedance of different positions of the operation site. It can be understood that the temperature acquisition circuit and the impedance acquisition circuit can be integrated together, or can be provided in separate modules.

Each syringe arrangement 23 includes a syringe 231, an extension tube 232, a push rod 233 and a driving device 234. Each syringe arrangement 23 forms a perfusion channel.

One end of each extension tube 232 is connected to the syringe, and the other end is provided with at least one temperature and impedance acquisition device 22 (for illustration, only one is shown in the figure). One end of the push rod 233 abuts against the syringe 231, and the push rod 233 is also connected with the driving device 234 (such as a stepping motor).

Optionally, the end of each extension tube 232 provided with the temperature and impedance acquisition device 22 may be fixed around the top 41 of the radio frequency operation catheter 40 through a fixing structure. The fixing structure is, for example, a conduit. The side wall of the conduit has a plurality of through holes, and has a plurality of through passages running through the front and rear ends of the conduit. The extension tubes 232 and the top 41 of the radio frequency operation catheter 40 respectively pass through the plurality of through passages, and the temperature and impedance acquisition devices 22 arranged at the end of the extension tubes 232 pass through the through passages along with the extension tubes 232 and then respectively pass through the through holes on the side wall of the conduit closest to itself, so that the temperature and impedance acquisition devices 22 together assume a claw structure for acquiring the temperature of different positions of the radio frequency operation object or operation site.

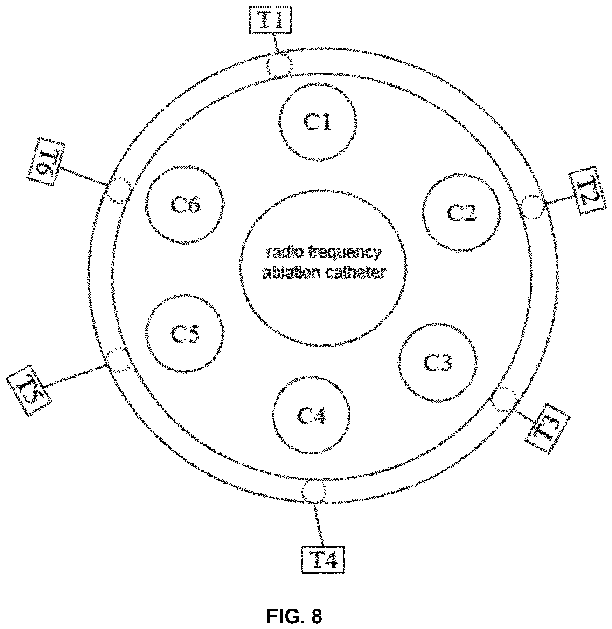

Taking six syringe arrangements as an example, as shown in FIG. 8, the dotted circles in the figure represent the through holes in the side wall of the conduit. The six syringe arrangements surround the radio frequency operation catheter to form six perfusion channels C1 to C6, and the six perfusion channels C1 to C6 correspond to six temperature acquisition devices T1 to T6 respectively. The radio frequency operation catheter may be a mono-pole radio frequency operation catheter or a multi-pole radio frequency operation catheter, which is not specifically limited in the present disclosure. In the case where the radiofrequency operation catheter is a multi-pole radiofrequency operation catheter, at least one perfusion channel can be provided around each pole of the multi-pole radiofrequency operation catheter, or one perfusion channel can be shared by multiple poles.

The controller 21 opens or closes the respective perfusion channel by controlling the driving device 234 to drive the push rod 233 to move in a specified direction or not. For example, when the push rod 233 pushed the rear end of the syringe 231 forward, the perfusion channel can be opened and thus the liquid in the syringe 231 can flow into the radio frequency operation object along the perfusion channel. When the push rod 233 stopped pushing the rear end of the syringe 231 forward, the perfusion channel is closed and thus the liquid in the syringe 231 can no longer flow into the radio frequency operation object along the perfusion channel.

Further, the controller 21 controls the flow rate of the liquid in the perfusion channel by controlling the movement speed of the push rod through the driving device 234.

Optionally, a valve can be provided on the extension tube 232 of each syringe arrangement, and the controller 21 opens or closes the respective perfusion channel by controlling the on / off of the valve.

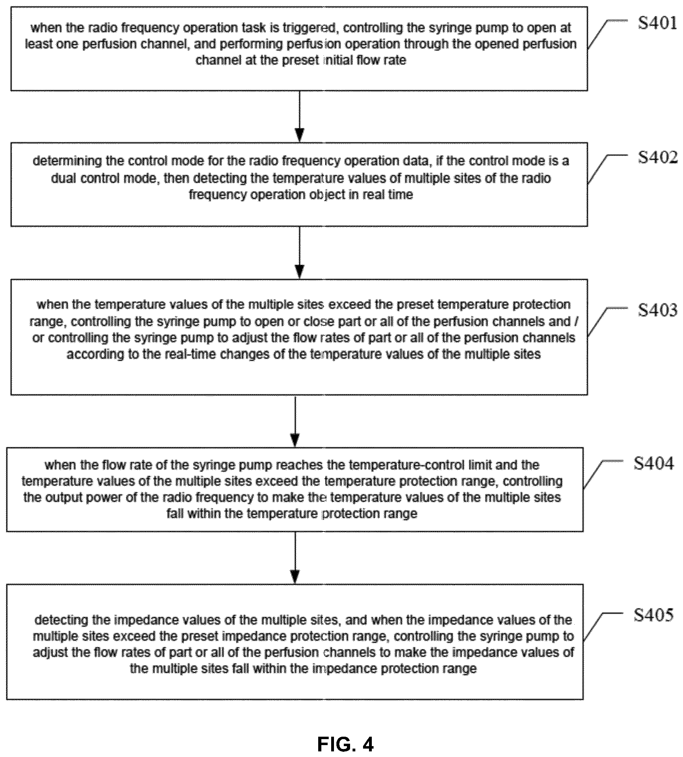

The controller 21 is electrically coupled with a plurality of temperature and impedance acquisition devices 22 through data lines or wireless networks, and is electrically connected with the multiple syringe arrangements 23 for executing the steps in the radio frequency operation data control methods according to the following embodiments shown in FIGS. 4 to 7. For example,

When the radio frequency operation task is triggered, controlling and opening at least one perfusion channel, and performing perfusion operation through the opened perfusion channel at the preset initial flow rate;

Determining the control mode for the radio frequency operation data, if the control mode is a dual control mode, then detecting the temperature values of multiple sites of the radio frequency operation object in real time;

When the temperature values of the multiple sites exceed the preset temperature protection range, controlling and opening or closing part or all of the perfusion channels, and / or controlling and adjusting the flow rates of part or all of the perfusion channels according to the real-time changes of the temperature values of the multiple sites;

When the flow rate of the syringe pump reaches the temperature-control limit and the temperature values of the multiple sites exceed the temperature protection range, controlling the output power of the radio frequency to make the temperature values of the multiple sites fall within the temperature protection range;

Detecting the impedance values of the multiple sites, and when the impedance values of the multiple sites exceed the preset impedance protection range, controlling and adjusting the flow rates of part or all of the perfusion channels to make the impedance values of the multiple sites fall within the impedance protection range.

The specific process for realizing the functions of the controller 21 can refer to the relevant description in the following embodiments shown in FIG. 4 to FIG. 7, which will not be repeated here.

It can be understood that the syringe pump 20 can include other common structures such as a display screen and a power supply, which are not specifically limited in the present disclosure.

In the embodiment of the present disclosure, by using multiple syringe arrangements, when the radio frequency operation task is triggered, the syringe pump is controlled to open and perform the perfusion operation through at least one perfusion channel at the preset initial flow rate, and the control mode for the radio frequency operation data is determined. In case of a dual control mode, the temperature of multiple different sites of the radio frequency operation object is acquired in real time, and the syringe pump is controlled to open or close part or all of the perfusion channels, and / or to adjust the flow rates of part or all of the perfusion channels to control the temperature; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature changes of multiple different sites of the radio frequency operation object. Since the perfusion volume of the syringe pump is adjusted more purposefully and directionally according to the real-time changes of the temperature values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

Referring to FIG. 4, a flowchart of a radio frequency operation data control method according to an embodiment of the present disclosure is shown. The method is used to control a syringe pump with multiple perfusion channels, such as the syringe pump 20 shown in FIGS. 2 and 3. Specifically, the method can be implemented by the syringe pump 20 in FIG. 1, or the radio frequency host 10 in FIG. 1, or other computer devices electrically coupled to the syringe pump. As shown in FIG. 4, the method specifically includes:

Step S401, when the radio frequency operation task is triggered, controlling the syringe pump to open at least one perfusion channel, and performing perfusion operation through the opened perfusion channel at the preset initial flow rate;

Specifically, the radio frequency operation task may be triggered when, for example, meeting a preset trigger time, receiving a trigger instruction sent by other computer device, or detecting a notification event that the user performs an operation for triggering the radio frequency operation task. The operation for triggering the radio frequency operation task is, for example, pressing a physical or virtual button for triggering the radio frequency operation task.

Optionally, after starting the syringe pump every time, the perfusion parameters are set to preset initial values. The perfusion parameters may include, but are not limited to, initial flow rate, total perfusion volume, perfusion time, and the like.

When the radio frequency operation task is triggered, the radio frequency operation catheter starts to perform radio frequency operation on the radio frequency operation object, so as to output radio frequency energy to the radio frequency operation object; and at the same time, the syringe pump responds to the triggered perfusion control instruction and opens at least one perfusion channel indicated by the perfusion control instruction, and performs the perfusion operation through the opened perfusion channel at the preset initial flow rate, to perfuse the liquid into the radio frequency operation object to adjust the temperature of the radio frequency operation object, avoiding burning the external tissue of the radio frequency operation object due to too high temperature, or avoiding unsatisfied operation effect due to too low temperature.

The perfusion control instruction can be automatically triggered by the syringe pump when detecting the preset event, or sent to the syringe pump by an ablation control device or other computer device electrically coupled to the syringe pump. The preset event includes an event that the user presses the preset physical or virtual key for triggering the perfusion control instruction, or an event that triggers the radio frequency operation task.

Step S402, determining the control mode for the radio frequency operation data, if the control mode is a dual control mode, then detecting the temperature values of multiple sites of the radio frequency operation object in real time;

Specifically, the temperatures of the multiple different sites of the radio frequency operation object are acquired in real time through a plurality of temperature acquisition devices.

The data control mode can be set by the user, and includes single control mode and dual control mode. The single control mode includes single, temperature control mode and single, impedance control mode, and the dual control mode is dual impedance-temperature control mode.

Specifically, the user's setting on the data control mode can be obtained if the user has set the data control mode, then the control mode for the radio frequency operation data is determined according to the user's setting; if the user has not set the data control mode, then the dual control mode is determined as the control mode for the radio frequency operation data.

The dual control mode is to control the temperature first, and then control the impedance after the temperature is controlled to the normal range. The single, temperature control mode is to only control the temperature. The single, impedance control mode is to only control the impedance. Alternatively, the single, temperature control mode is to control the radio frequency operation data according to the change of the temperature value. The single, impedance control mode is to control the radio frequency operation data according to the change of the impedance value.

Optionally, the method of controlling the radio frequency operation data according to the change of the temperature value can refer to the relevant description of the following steps S403 and S404, which will not be repeated here.

Optionally, as the working principle of controlling the radio frequency operation data according to the change of the impedance value is similar to that of controlling the radio frequency operation data according to the change of the temperature value, except that the reference standard of the control is impedance instead of the temperature, the detail can also refer to the following step S403 and S404, which will not be repeated here.

Step S403, when the temperature values of the multiple sites exceed the preset temperature protection range, controlling the syringe pump to open or close part or all of the perfusion channels and / or controlling the syringe pump to adjust the flow rates of part or all of the perfusion channels according to the real-time changes of the temperature values of the multiple sites;

Specifically, a plurality of temperature acquisition devices are configured in the radio frequency operating system to respectively acquire the temperatures of multiple different sites of the radio frequency operation object. The multiple perfusion channels of the syringe pump are respectively configured to perfuse liquids to different sites of the radio frequency operation object. When the perfusion channels are opened, the liquid will automatically flow to the corresponding sites through the opened perfusion channels. One perfusion channel is provided with at least one temperature acquisition device.

The temperature values of the multiple sites exceeding the preset temperature protection range means that the temperature value of at least one of the multiple sites is greater than the maximum value of the temperature protection range, or smaller than the minimum value of the temperature protection range.

According to the temperatures of the multiple sites acquired in real time, the real-time changes of the temperature values of the multiple sites can be analyzed, and when the real-time change trend and change range of the multiple temperature values meet the preset adjustment condition, the syringe pump is controlled to turn on or off part or all of the perfusion channels, and / or, the syringe pump is controlled to adjust the flow rates of part or all of the perfusion channels.

The preset adjustment condition is that, for example, the acquired temperature is greater than the preset maximum temperature, or the acquired temperature is lower than the preset minimum temperature, or the like.

Controlling the syringe pump to open or close part or all of the perfusion channels and / or controlling the syringe pump to adjust the flow rates of part or all of the perfusion channels means controlling the syringe pump to perform at least one of the following operations:

Controlling the syringe pump to open some perfusion channels; controlling the syringe pump to close some perfusion channels; controlling the syringe pump to open all perfusion channels; controlling the syringe pump to close all perfusion channels; controlling the syringe pump to adjust the flow rate of some perfusion channels; and controlling the syringe pump to adjust the flow rates of all perfusion channels.

The flow rate of the perfusion channel is the flow rate of the liquid in the perfusion channel. By controlling the flow rate of the liquid in the perfusion channel, the perfusion volume of the perfusion channel can be controlled, and thus the temperature of the radio frequency operation object can be controlled.

Optionally, after opening the perfusion channel, the syringe pump can automatically perform the perfusion operation directly through the opened perfusion channel.

Step S404, when the flow rate of the syringe pump reaches the temperature-control limit and the temperature values of the multiple sites exceed the temperature protection range, controlling the output power of the radio frequency to make the temperature values of the multiple sites fall within the temperature protection range;

The temperature-control limit corresponding to the flow rate of the liquid of the syringe pump is preset. When it is detected that the current flow rate has reached the temperature-control limit, if the temperature values of the multiple sites still exceed the temperature protection range, the temperature will not be controlled by changing the flow rate. Instead, the temperature will be further controlled by controlling and adjusting the output power of the radio frequency. Controlling the temperature values of the multiple sites to be within the temperature protection range can avoid other physical damage to the radio frequency operation object due to excessive liquid perfusion. The output power of the radio frequency is the output power of the radio frequency host.

Step S405: detecting the impedance values of the multiple sites, and when the impedance values of the multiple sites exceed the preset impedance protection range, controlling the syringe pump to adjust the flow rates of part or all of the perfusion channels to make the impedance values of the multiple sites fall within the impedance protection range.

When the temperature is controlled to a normal value, that is, when it falls back within the temperature protection range, the impedance values of the multiple sites are detected. When the impedance values of the multiple sites exceed the preset impedance protection range, the syringe pump is controlled to adjust the flow rates of part or all of the perfusion channels to make the impedance values of the multiple sites fall within the impedance protection range. Adjusting the impedance values by controlling the syringe pump to adjust the flow rates of part or all of the perfusion channels can refer to the aforementioned description of adjusting the temperature values by controlling the syringe pump to adjust the flow rates of part or all of the perfusion channels.

In the embodiment of the present disclosure, by using multiple syringe arrangements, when the radio frequency operation task is triggered, the syringe pump is controlled to open and perform the perfusion operation through at least one perfusion channel at the preset initial flow rate, and the control mode for the radio frequency operation data is determined. In case of a dual control mode, the temperature of multiple different sites of the radio frequency operation object is acquired in real time, and the syringe pump is controlled to open or close part or all of the perfusion channels, and / or to adjust the flow rates of part or all of the perfusion channels to control the temperature; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature and impedance changes of multiple different sites of the radio frequency operation object. Since the perfusion volume of the syringe pump is adjusted more purposefully and directionally according to the real-time changes of the temperature and impedance values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

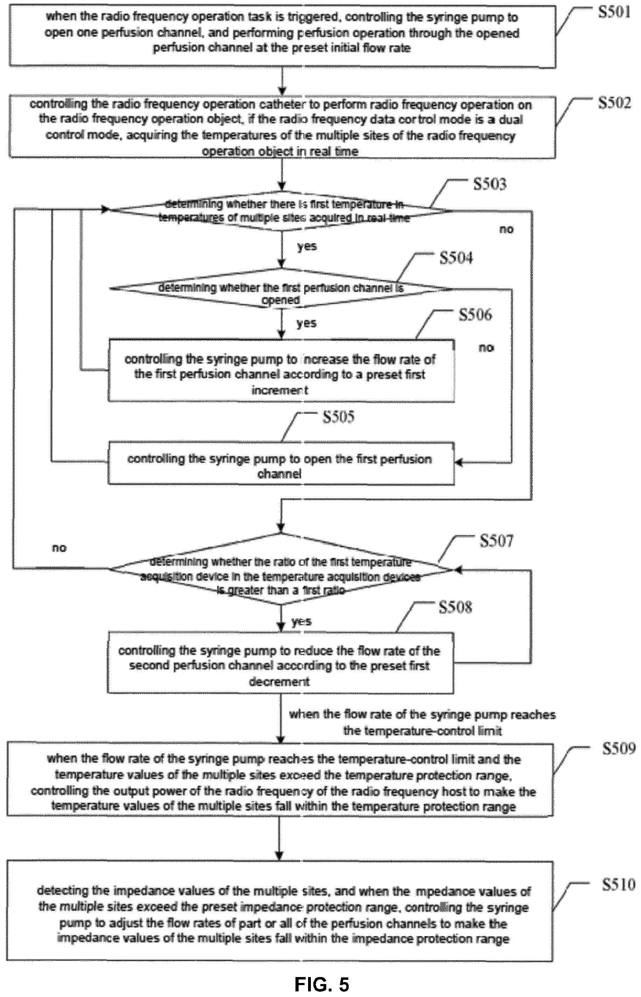

Referring to FIG. 5, a flowchart of a radio frequency operation data control method according to another embodiment of the present disclosure is shown. The method is used to control a syringe pump with multiple perfusion channels, such as the syringe pump 20 shown in FIGS. 2 and 3. Specifically, the method can be implemented by the syringe pump 20 in FIG. 1, or the radio frequency host 10 in FIG. 1, or other computer devices electrically coupled to the syringe pump, for the convenience of description, which are all referred to as the control apparatus in the following. As shown in FIG. 5, the method specifically includes:
Step S501, when the radio frequency operation task is triggered, controlling the syringe pump to open one perfusion channel, and performing perfusion operation through the opened perfusion channel at the preset initial flow rate;
Step S502, controlling the radio frequency operation catheter to perform radio frequency operation on the radio frequency operation object, if the radio frequency data control mode is a dual control mode, acquiring the temperatures of the multiple sites of the radio frequency operation object in real time;
Specifically, the radio frequency operation catheter is controlled to perform radio frequency operation on the radio frequency operation object, and the control mode for the radio frequency operation data is determined; if the radio frequency data control mode is the dual control mode, the temperatures of the multiple sites of the radio frequency operation object are acquired in real time through a plurality of temperature acquisition devices.

When the radio frequency operation task is triggered, the control apparatus first controls the syringe pump to randomly open one perfusion channel, so as to perfuse the liquid through the opened perfusion channel to the radio frequency operation object at the initial flow rate, then controls the radio frequency operation catheter to perform radio frequency operation on the radio frequency operation object, and at the same time, acquires the temperatures of the multiple sites of the radio frequency operation object in real time through the plurality of temperature acquisition devices.

Before controlling the radio frequency operation catheter to perform radio frequency operation, first controlling the syringe pump to randomly open a perfusion channel, through which a small amount of liquid is perfused to the radio frequency operation object, can avoid affecting the following control operations based on the impedance values due to individual difference of the radio frequency operation object with too high initial impedance, and the perfusion with the small amount of liquid will not cause other adverse effects on the radio frequency operation object.

The details of step S501 and step S502 in this embodiment can refer to the relevant description of step S401 and step S402 in the embodiment shown in FIG. 4.

Step S503, determining whether there is a first temperature in the temperatures of the multiple sites acquired in real time;
The first temperature is greater than the preset maximum temperature. Optionally, the preset maximum temperature can be preset in the execution body of the radio frequency operation data control method herein as user defined.

If there is the first temperature, step S504 of determining whether the first perfusion channel is opened is executed;
Specifically, if there is the first temperature in the temperatures of the multiple sites acquired in real time, it means that the temperatures of some sites of the radio frequency operation object exceed the limit, and there is a risk of damage and it is necessary to increase the perfusion volume to lower the temperature, and thus it is determined whether the first perfusion channel is opened. The first perfusion channel is configured to perfuse liquid into the first site, and the first temperature is the temperature of the first site.

For example, provided that there are four temperature acquisition devices T1 to T4 respectively configured to acquire the temperatures of four sites B1 to B4 of the radio frequency operation object, and respectively corresponding to four perfusion channels C1 to C4 of the syringe pump, the preset maximum temperature is 90 °C (Celsius), if the temperatures acquired by the four temperature acquisition devices T1 to T4 are 89.7 °C, 91 °C, 89.9 °C and 90.5 °C respectively, according to the correspondence among the temperature acquisition devices, operation sites, perfusion channels and temperatures acquired in real time of {(T1, B1, C1, 89.7 °C), (T2, B2, C2, 91 °C), (T3, B3, C3, 89.9 °C), (T4, B4, C4, 90.5 °C)} and the preset maximum temperature of 90 °C, it can be determined that the first temperatures are 91 °C and 90.5 °C that exceed the limit, the first sites that need to be cooled are B2 and B4, and the first perfusion channels that need to be controlled are C2 and C4.

Since the control apparatus has controlled the syringe pump to randomly open one perfusion channel when the radio frequency operation task was triggered, it needs to determine whether the first perfusion channels C2 and C4 have been opened.

The control apparatus stores identification information (for example, serial number) of the multiple perfusion channels of the syringe pump, and each time the control apparatus controls the syringe pump to open or close the perfusion channel, it will generate a corresponding log, which records at least the identification information, the flow rate, and the opening or closing time of the perfusion channel that is opened or closed currently. According to the log, the currently opened perfusion channel can be determined, so that it can be determined whether the first perfusion channels C2 and C4 have been opened.

If the first perfusion channel is not opened, step S505 of controlling the syringe pump to open the first perfusion channel is executed, and return to step S503;
If the first perfusion channel is opened, step S506 of controlling the syringe pump to increase the flow rate of the first perfusion channel according to a preset first increment is executed, and return to step S503 until the flow rate of the first perfusion channel reaches the preset temperature-control flow rate limit;
Specifically, on the one hand, if the first perfusion channel is not opened, the syringe pump is controlled to open the first perfusion channel, so as to perform the perfusion operation through all the first perfusion channels that are opened, and return to the step of determining whether there is the first temperature in the temperatures of the multiple sites acquired in real time; on the other hand, if the first perfusion channel has been opened, then the syringe pump is controlled to increase the flow rate of the first perfusion channel according to the preset first increment, and return to the step of determining whether there is the first temperature in the temperatures of the multiple sites acquired in real time, and so on until the flow rates of all the first perfusion channels reach the preset temperature-control flow rate limit.

Following the above example, if C2 of the first perfusion channels C2 and C4 has been opened while C4 is not opened, then the syringe pump is controlled to open C4, and the liquid is perfused to site B4 through C4 at the initial flow rate; at the same time, the syringe pump is controlled to increase the flow rate of C2 according to the preset first increment to increase the perfusion volume of site B2, thereby cooling sites B2 and B4 rapidly.

If there is no first temperature, step S507 of determining whether the ratio of the first temperature acquisition device in the temperature acquisition devices is greater than a first ratio is executed;
Specifically, if there is no first temperature higher than the preset maximum temperature in the temperatures of the multiple sites acquired in real time, it means that the temperatures of all sites of the radio frequency operation object are within the safe range, and the current radio frequency operation will not cause damage to the radio frequency operation object, and thus it is determined whether the ratio of the first temperature acquisition device in the temperature acquisition devices is greater than the first ratio, so as to ensure that the radio frequency operation can achieve the expected operation effect.

The temperature acquired by the first temperature acquisition device is less than the preset minimum temperature for a preset time period, and the preset minimum temperature is the lowest temperature limit for achieving the expected operation effect. Optionally, the preset minimum temperature, preset time period and first ratio can be preset in the execution body of the radio frequency operation data control method here as user defined.

If the ratio is greater than the first ratio, step S508 of controlling the syringe pump to reduce the flow rate of the second perfusion channel according to the preset first decrement is executed, and return to step S507 until the flow rate of the second perfusion channel reaches the preset minimum flow rate;
If the ratio is not greater than the first ratio, return to step S503.

Specifically, on the one hand, if the ratio of the first temperature acquisition device in the temperature acquisition devices is greater than the first ratio, it means that the overall temperature of the radio frequency operation object is low, with a slow temperature rise, and the current liquid perfusion volume is too large, which may result in unsatisfied operation effect, then the syringe pump is controlled to reduce the flow rate of the second perfusion channel according to the preset first decrement, and return to the step of determining whether the ratio of the first temperature acquisition device in the temperature acquisition devices is greater than the first ratio, and so on until the flow rate of the second perfusion channel reaches the preset minimum flow rate. The second perfusion channel is configured to perfuse liquid to the second site, and the first temperature acquisition device is configured to detect the temperature of the second site.

On the other hand, if the ratio of the first temperature acquisition device in the temperature acquisition devices is not greater than the first ratio, it means that the temperature rise of the radio frequency operation object is positive, and the current perfusion volume facilitates the temperature rise of the radio frequency operation object, so return to execute the step of determining whether there is the first temperature in the temperatures of the multiple sites acquired in real time.

According to the real-time changes of the temperature value, gradually increasing or decreasing the perfusion volume can improve the accuracy of perfusion control, reduce operational risks, and achieve better operation effect.

Following the above example, provided that the preset minimum temperature is 65 °C (Celsius), the first ratio is 49%, and the preset time period is 10 seconds, if the temperatures acquired by the four temperature acquisition devices T1 to T4 are respectively 64.2 °C (for 11 seconds), 64.3 °C (for 9 seconds), 65.1 °C (for 6 seconds) and 64.2 °C (for 12 seconds), according to the correspondence among the temperature acquisition devices, operation sites, perfusion channels and temperatures acquired in real time of {(T1, B1, C1, 64.2 °C, 11 seconds ), (T2, B2, C2, 64.3 °C, 9 seconds ), (T3, B3, C3, 65.1 °C, 6 seconds ), (T4, B4, C4, 64.2 °C, 12 seconds )} and the preset minimum temperature of 65 °C, it can be determined that the first temperature acquisition devices are T1 and T4, and the ratio of the first temperature acquisition devices in the temperature acquisition devices is 2 / 4 = 50% (greater than the first ratio of 49%), the second perfusion channels that need to be controlled are C1 and C4. Therefore, the syringe pump is controlled to reduce the flow rates of C1 and C4 according to the preset first decrement, so as to reduce the perfusion volume to the sites B1 and B4, thereby achieving the effect of increasing the temperature of the sites B1 and B4.

Optionally, in another embodiment of the present disclosure, respective preset maximum and minimum temperatures are set for each temperature acquisition device, and the above-mentioned first and second perfusion channels are determined based on the respective preset maximum and minimum temperatures of the temperature acquisition devices. Then, the above steps S503 to S508 are executed according to the first perfusion channel and the second perfusion channel determined based on the respective preset maximum temperatures of the respective temperature acquisition devices.

Specifically, it is determined whether there is the first temperature in the temperatures of the multiple sites acquired in real time, and the first temperature is greater than the preset maximum temperature of the temperature acquisition device that acquires the first temperature.

On the one hand, if there is the first temperature (the temperature of the first site) in the temperatures of the multiple sites acquired in real time, it is determined whether the first perfusion channel for liquid perfusion to the first site is opened; if the first perfusion channel is not opened, then the syringe pump is controlled to open the first perfusion channel, and return to the step of determining whether there is the first temperature in the temperatures of the multiple sites acquired in real time, the first temperature being greater than the preset maximum temperature of the temperature acquisition device that acquires the first temperature; if the first perfusion channel has been opened, then the syringe pump is controlled to increase the flow rate of the first perfusion channel according to the preset first increment, and return to the step of determining whether there is the first temperature in the temperatures of the multiple sites acquired in real time, the first temperature being greater than the preset maximum temperature of the temperature acquisition device that acquires the first temperature, until the flow rate of the first perfusion channel reaches the preset temperature-control flow rate limit.

On the other hand, if there is no first temperature in the temperatures of the multiple sites acquired in real time, it is determined whether the ratio of the first temperature acquisition device in the temperature acquisition devices is greater than the first ratio, and the temperature acquired by the first temperature acquisition device is less than the preset minimum temperature corresponding to the first temperature acquisition device for a preset time period; if the ratio of the first temperature acquisition device is greater than the first ratio, the syringe pump is controlled to reduce the flow rate of the second perfusion channel according to the preset first decrement, and return to the step of determining whether the ratio of the first temperature acquisition device in the temperature acquisition devices is greater than the first ratio, until the flow rate of the second perfusion channel reaches the preset minimum flow rate, the second perfusion channel is configured to perfuse liquid into the second site, and the first temperature acquisition device is configured to detect the temperature of the second site; if the ratio of the first temperature acquisition device is not greater than the first ratio, then return to the step of determining whether there is the first temperature in the temperatures of the multiple sites acquired in real time, the first temperature being greater than the preset maximum temperature of the temperature acquisition device that acquires the first temperature.

Since different sites of the radio frequency operation object may have certain differences in surface shape, internal tissue structure and tissue thickness, the temperature changes caused by the influence of radio frequency energy are also different, and the temperature limits required for metamorphosis are also different. Presetting the respective maximum and minimum limits for temperature acquisition devices for acquiring the temperatures of different sites of the radio frequency operation object can improve the pertinence of the perfusion control, thereby further improving the accuracy of the perfusion operation and thus the operation effect.

Step S509, when the flow rate of the syringe pump reaches the temperature-control limit and the temperature values of the multiple sites exceed the temperature protection range, controlling the output power of the radio frequency of the radio frequency host to make the temperature values of the multiple sites fall within the temperature protection range;
In the above step, the radio frequency operation object is controlled by adjusting the flow rate of the perfusion liquid. When the current flow rate reaches the temperature-control limit, if the temperature values of the multiple sites exceed the temperature protection range, the temperature will not be controlled by changing the flow rate. Instead, the temperature will be further controlled by controlling the output power of the radio frequency of the radio frequency host to adjust the temperature values of the multiple sites to be within the temperature protection range.

Step S510, detecting the impedance values of the multiple sites, and when the impedance values of the multiple sites exceed the preset impedance protection range, controlling the syringe pump to adjust the flow rates of part or all of the perfusion channels to make the impedance values of the multiple sites fall within the impedance protection range.

When the temperature is controlled to a normal value, that is, when it falls back within the temperature protection range, the impedance values of the multiple sites are respectively detected by one or more impedance detection devices. When the impedance values of the multiple sites exceed the preset impedance protection range, the syringe pump is controlled to adjust the flow rates of part or all of the perfusion channels to make the impedance values of the multiple sites fall within the impedance protection range.

In the embodiment of the present disclosure, when the radio frequency operation task is triggered, the syringe pump is controlled to randomly open one perfusion channel, and perform the perfusion operation through the opened perfusion channel at the preset initial flow rate, and the control mode for the radio frequency operation data is determined. In case of a dual control mode, according to the temperatures of multiple different sites of the radio frequency operation object acquired in real time by the plurality of temperature acquisition devices, the syringe pump is controlled to open or close part or all of the perfusion channels and / or to adjust the flow rates of part or all of the perfusion channels; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature and impedance changes of multiple different sites of the radio frequency operation object. Since the perfusion volume of the syringe pump is adjusted more purposefully and directionally according to the real-time changes of the temperature and impedance values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

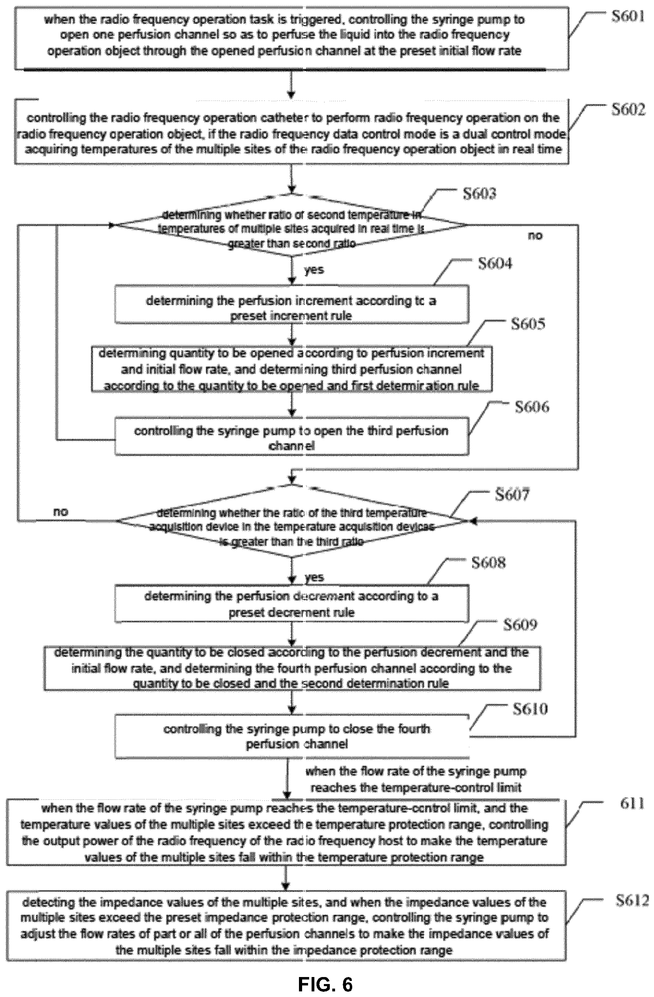

Referring to FIG. 6, a flowchart of a radio frequency operation data control method according to a further embodiment of the present disclosure is shown. The method is used to control a syringe pump with multiple perfusion channels, such as the syringe pump 20 shown in FIGS. 2 and 3. Specifically, the method can be implemented by the syringe pump 20 in FIG. 1, or the radio frequency host 10 in FIG. 1, or other computer devices electrically coupled to the syringe pump, for the convenience of description, which are all referred to as the control apparatus in the following. As shown in FIG. 6, the method specifically includes:
Step S601, when the radio frequency operation task is triggered, controlling the syringe pump to open one perfusion channel so as to perfuse the liquid into the radio frequency operation object through the opened perfusion channel at the preset initial flow rate;
Step S602, controlling the radio frequency operation catheter to perform radio frequency operation on the radio frequency operation object, if the radio frequency data control mode is a dual control mode, acquiring the temperatures of the multiple sites of the radio frequency operation object in real time;
The radio frequency operation catheter is controlled to perform radio frequency operation on the radio frequency operation object, and the control mode for the radio frequency operation data is determined; if the radio frequency data control mode is the dual control mode, the temperatures of the multiple sites of the radio frequency operation object are acquired in real time through a plurality of temperature acquisition devices.

Step S601 and step S602 are the same as step S501 and step S502 in the embodiment shown in FIG. 5, the details refer to the relevant description in the embodiment shown in FIG. 5, which will not be repeated here.

Step S603, determining whether the ratio of the second temperature in the temperatures of the multiple sites acquired in real time is greater than the second ratio;
The second temperature is greater than the preset maximum temperature. The second ratio can be preset in the execution body of the radio frequency operation data control method herein as user defined.

If the ratio of the second temperature is greater than the second ratio, step S604 of determining the perfusion increment according to a preset increment rule is executed;
Specifically, if the ratio of the second temperature that is greater than the preset maximum temperature in the temperatures of the multiple sites acquired in real time is greater than the second ratio, it means that the temperature of the radio frequency operation object is generally high, and there is a risk of damage and it is necessary to increase the perfusion volume to lower the temperature, and thus the perfusion increment (that is, the increased perfusion volume required) is determined according to the preset increment rule.

The preset increment rule is to determine the perfusion increment according to the difference between the maximum temperature in the temperatures of the multiple sites and the preset maximum temperature. The difference between the maximum temperature in the temperatures of the multiple sites and the preset maximum temperature is directly proportional to the perfusion increment, that is, the larger the difference, the greater the required perfusion increment.

Optionally, the perfusion increment and the perfusion decrement hereinafter may be fixed values preset in the control apparatus as user defined.

Specifically, the correspondence between multiple difference ranges and preset perfusion increments can be preset in the control apparatus. First, which difference range the maximum temperature in the temperatures of the multiple sites falls into is determined, and then the perfusion increment corresponding to the difference range fallen into is determined as the required perfusion increment according to the difference range fallen into and the above-mentioned preset correspondence.

Step S605, determining the quantity of the perfusion channels to be opened according to the perfusion increment and the initial flow rate, and determining the third perfusion channel according to the quantity of the perfusion channels to be opened and the first determination rule;

It can be understood that the initial flow rate of the perfusion channels is the same. At the beginning of controlling and opening the perfusion channel by the syringe pump, the perfusion operation will be performed through the opened perfusion channel at the initial flow rate.

Specifically, the first determination rule is to determine the third perfusion channel according to the distance from the second temperature acquisition device and the quantity of the perfusion channels to be opened in order from proximal to distal. The temperature acquired by the second temperature acquisition device is the maximum temperature. Further, if there are multiple perfusion channels at the same distance from the second temperature acquisition device, the required third perfusion channel is randomly determined among them.

The calculation method for determining the quantity of the perfusion channels to be opened according to the perfusion increment and the initial flow rate is, for example, dividing the perfusion increment by the initial flow rate, rounding the obtained value and then plus 1.

For example, referring to FIG. 8, provided that there are six temperature acquisition devices T1 to T6 corresponding to the six sites B1 to B6 of the radio frequency operation object, and six perfusion channels C1 to C6 of the syringe pump are configured to perfuse liquid to sites B1 to B6 respectively. If the preset maximum temperature is 90 °C, the second ratio is 50%, and the temperatures of sites B1 to B6 acquired in real time by T1 to T6 are 90.2 °C, 80.9 °C, 90.1 °C, 90.3 °C, 80.8 °C, and 90.3 °C respectively, it can be determined that there are four second temperatures, namely 90.2 °C, 90.1 °C, 90.3 °C, and 90.3 °C and the ratio of the second temperatures in all the temperatures acquired by the six temperature acquisition devices T1 to T6 is 4 / 6 = 67%, which is greater than the second ratio of 50%. Therefore, according to the difference of 0.3 °C between the maximum temperature of 90.3 °C among the six temperatures and the preset maximum temperature of 90 °C and the correspondence between the preset multiple difference ranges and the preset perfusion increments, the required perfusion increment can be determined, for example, 0.5 ml. Then, according to the initial flow rate of each perfusion channel (for example, 0.2 ml) and the determined perfusion increment, the quantity of the perfusion channels to be opened can be determined as [(0.5 / 0.2)] + 1 = 3. Finally, according to the distance from the second temperature acquisition device T6 (acquiring the maximum temperature) and the quantity of the perfusion channels to be opened, the third perfusion channels to be opened can be determined as C5, C1, and C2 in order from proximal to distal.

Step S606, controlling the syringe pump to open the third perfusion channel, and return to step S603 until all perfusion channels are opened;
The control apparatus stores identification information of the multiple perfusion channels of the syringe pump. According to the identification information, the syringe pump is controlled to open the third perfusion channel, and return to the step of determining whether the ratio of the second temperature in the temperatures of the multiple sites acquired in real time is greater than the second ratio until all perfusion channels are opened.

Further, each time the control apparatus controls the syringe pump to open or close the perfusion channel, it will generate a corresponding log, which records at least the identification information, the flow rate and the opening or closing time of the perfusion channel that is opened or closed currently. Before controlling the syringe pump to open the third perfusion channel, it can be determined according to the log whether the third perfusion channel has been opened. If not opened, then the third perfusion channel can be opened. If opened, the third perfusion channel can be bypassed, and the adjacent perfusion channel can be opened. Following the above example, if C2 has been opened, then the perfusion channel C3 adjacent to C2 can be opened. It can be understood that if C3 is also opened, the perfusion channel C4 is opened sequentially, and so on, until all the perfusion channels are opened, or the quantity of the opened third perfusion channels reaches the quantity of the perfusion channels to be opened.

Further, after opening all the perfusion channels, if the ratio of the second temperature is still greater than the second ratio, it means that the previous perfusion effect is unsatisfied, and the overall temperature of the radio frequency operation object is still too high, then the syringe pump can be controlled to increase the flow rates of all perfusion channels from the initial flow rate to the preset temperature-control flow rate limit at one time so as to achieve the effect of rapid cooling.

If the ratio of the second temperature is not greater than the second ratio, step S607 of determining whether the ratio of the third temperature acquisition device in the temperature acquisition devices is greater than the third ratio is executed;

Specifically, if the ratio of the second temperature greater than the preset maximum temperature in the temperatures of the multiple sites acquired in real time is not greater than the second ratio, it means that the overall temperature of the radio frequency operation object falls within the safe range, and the current radio frequency operation will not cause damage to the radio frequency operation object, and thus it is determined whether the ratio of the third temperature acquisition device in the temperature acquisition devices is greater than the third ratio, so as to ensure that the radio frequency operation can achieve the expected operation effect. The temperature acquired by the third temperature acquisition device is lower than the preset minimum temperature. The third ratio can be preset in the execution body of the radio frequency operation data control method herein as user defined.

If the ratio of the third temperature acquisition device is greater than the third ratio, step S608 of determining the perfusion decrement according to a preset decrement rule is executed;
If the ratio of the third temperature acquisition device in all temperature acquisition devices is greater than the third ratio, it means that the overall temperature of the radio frequency operation object is low, and the current liquid perfusion volume is too large, which may result in unsatisfied operation effect, then the perfusion decrement can be determined according to the preset decrement rule.

The preset decrement rule is to determine the perfusion decrement according to the difference between the lowest temperature in the temperatures of the multiple sites and the preset minimum temperature. The difference between the minimum temperature and the preset minimum temperature is directly proportional to the perfusion decrement, that is, the larger the difference, the greater the perfusion decrement required.

Specifically, the correspondence between multiple difference ranges and preset perfusion decrements can be preset in the control apparatus. First, which difference range the lowest temperature in the temperatures of the multiple sites falls into is determined, and then the perfusion decrement corresponding to the difference range fallen into is determined as the required perfusion decrement according to the difference range fallen into and the above-mentioned preset correspondence.

Step S609, determining the quantity of the perfusion channels to be closed according to the perfusion decrement and the initial flow rate, and determining the fourth perfusion channel according to the quantity of the perfusion channels to be closed and the second determination rule;
Specifically, the calculation method for determining the quantity of the perfusion channels to be closed according to the perfusion decrement and the initial flow rate is, for example, dividing the perfusion decrement by the initial flow rate, rounding the obtained value and then plus 1.

The second determination rule is to determine the fourth perfusion channel according to the distance from the fourth temperature acquisition device in order from proximal to distal and the quantity of the perfusion channels to be closed. The temperature acquired by the fourth temperature acquisition device is the lowest.

The method for determining the fourth perfusion channel is similar to that for the third perfusion channel, and the details refer to the related description in step S605, which will not be repeated here.

Step S610, controlling the syringe pump to close the fourth perfusion channel, and return to step S607 until all perfusion channels are closed;
The control apparatus controls the syringe pump to close the fourth perfusion channel according to the identification information of the perfusion channels, and returns to the step of determining whether the ratio of the third temperature acquisition device in the temperature acquisition devices is greater than the third ratio, until all perfusion channels are closed.

Further, if the fourth perfusion channel to be closed has been closed, then close the perfusion channel adjacent to the fourth perfusion channel; and if the adjacent perfusion channel has also been closed, then close the next adjacent perfusion channel adjacent to the adjacent fourth perfusion channel in order, and so on, until all perfusion channels are closed, or the quantity of the closed fourth perfusion channels reaches the quantity of the perfusion channels to be closed.

If the ratio of the third temperature acquisition device in all the temperature acquisition devices is not greater than the third ratio, return to step S603 of determining whether the ratio of the second temperature in the temperatures of the multiple sites acquired in real time is greater than the second ratio.

According to the real-time changes of the temperature value, gradually increasing or decreasing the perfusion volume can improve the accuracy of perfusion control, reduce operational risks, and achieve better operation effect.

Step S611, when the flow rate of the syringe pump reaches the temperature-control limit, and the temperature values of the multiple sites exceed the temperature protection range, controlling the output power of the radio frequency of the radio frequency host to make the temperature values of the multiple sites fall within the temperature protection range;
In the above step, the radio frequency operation object is controlled by adjusting the flow rate of the perfusion liquid. When the current flow rate reaches the temperature-control limit, the temperature will not be controlled by changing the flow rate. Instead, the temperature will be further controlled by controlling the output power of the radio frequency to adjust the temperature values of the multiple sites to be within the temperature protection range.

Step S612, detecting the impedance values of the multiple sites, and when the impedance values of the multiple sites exceed the preset impedance protection range, controlling the syringe pump to adjust the flow rates of part or all of the perfusion channels to make the impedance values of the multiple sites fall within the impedance protection range.

When the temperature is controlled to a normal value, that is, when it falls back within the temperature protection range, the impedance values of the multiple sites are detected. When the impedance values of the multiple sites exceed the preset impedance protection range, the syringe pump is controlled to adjust the flow rates of part or all of the perfusion channels to make the impedance values of the multiple sites fall within the impedance protection range. Adjusting the impedance values by controlling the syringe pump to adjust the flow rates of part or all of the perfusion channels can refer to the aforementioned description of adjusting the temperature values by controlling the syringe pump to adjust the flow rates of part or all of the perfusion channels.

The details not described here can refer to the relevant description in the embodiments shown in FIG. 4 and FIG. 5.

In the embodiment of the present disclosure, when the radio frequency operation task is triggered, the syringe pump is controlled to randomly open one perfusion channel, and perform the perfusion operation through the opened perfusion channel at the preset initial flow rate, and the control mode for the radio frequency operation data is determined. In case of a dual control mode, according to the temperatures of multiple different sites of the radio frequency operation object acquired in real time by the plurality of temperature acquisition devices, the syringe pump is controlled to open or close part or all of the perfusion channels and / or to adjust the flow rates of part or all of the perfusion channels; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature and impedance changes of multiple different sites of the radio frequency operation object. Since the perfusion volume of the syringe pump is adjusted more purposefully and directionally according to the real-time changes of the temperature and impedance values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

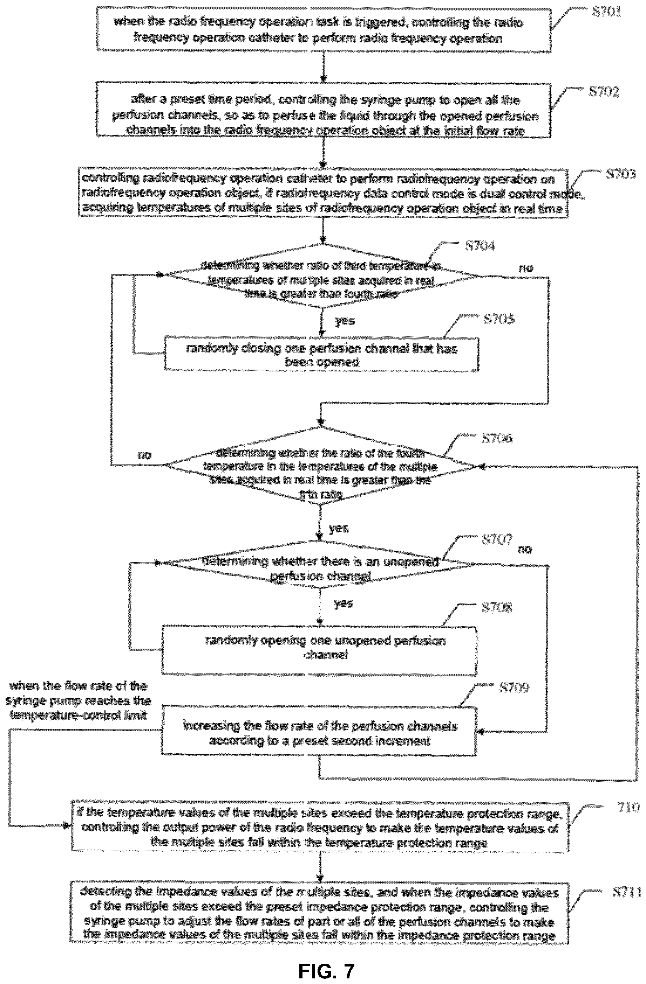

Referring to FIG. 7, a flowchart of a radio frequency operation data control method according to a still further embodiment of the present disclosure is shown. The method is used to control a syringe pump with multiple perfusion channels, such as the syringe pump 20 shown in FIGS. 2 and 3. Specifically, the method can be implemented by the syringe pump 20 in FIG. 1, or the radio frequency host 10 in FIG. 1, or other computer devices electrically coupled to the syringe pump, for the convenience of description, which are all referred to as the control apparatus in the following. As shown in FIG. 7, the method specifically includes:
Step S701, when the radio frequency operation task is triggered, controlling the radio frequency operation catheter to perform radio frequency operation;
Specifically, the radio frequency operation task may be triggered when, for example, meeting a preset trigger time, receiving a trigger instruction sent by other computer device, or detecting a notification event that the user performs an operation for triggering the radio frequency operation task. The operation for triggering the radio frequency operation task is, for example, pressing a physical or virtual button for triggering the radio frequency operation task.

Optionally, after starting the syringe pump every time, the perfusion parameters are set to preset initial values. The perfusion parameters may include, but are not limited to, initial flow rate, total perfusion volume, perfusion time, and the like.

When the radio frequency operation task is triggered, the radio frequency operation catheter starts to perform radio frequency operation on the radio frequency operation object, so as to output radio frequency energy to the radio frequency operation object.

Step S702, after a preset time period, controlling the syringe pump to open all the perfusion channels, so as to perfuse the liquid through the opened perfusion channels into the radio frequency operation object at the initial flow rate;
Specifically, the preset time period can be preset in the execution body of the radio frequency operation data control method herein as user defined.

It can be understood that the radio frequency operation object needs a certain temperature for achieving the expected operation effect. After controlling the radio frequency operation catheter to perform radio frequency operation and waiting for a preset time period, when the temperature of the radio frequency operation object rises to a certain level, then the syringe pump is controlled to open all the perfusion channels to perform the perfusion operation, avoiding affecting the temperature rise of the radio frequency operation object due to premature perfusion, and ensuring better operation effect.

Step S703, controlling the radio frequency operation catheter to perform radio frequency operation on the radio frequency operation object, if the radio frequency data control mode is a dual control mode, acquiring the temperatures of the multiple sites of the radio frequency operation object in real time;
Determine the control mode for the radio frequency operation data. If the radio frequency data control mode is a dual control mode, then the temperatures of the multiple sites of the radio frequency operation object can be acquired in real time through a plurality of temperature acquisition devices;
The details of step S703 can refer to the related description of step S402 in the embodiment shown in FIG. 4, which will not be repeated here.

Step S704, determining whether the ratio of the third temperature in the temperatures of the multiple sites acquired in real time is greater than the fourth ratio;
If the ratio of the third temperature is greater than the fourth ratio, step S705 of randomly closing one perfusion channel that has been opened is executed, and return to step S704;
Specifically, the third temperature is lower than the preset minimum temperature. If the ratio of the third temperature that is lower than the preset minimum temperature in the temperatures of the multiple sites acquired in real time is greater than the fourth ratio, it means that the current perfusion volume is too large, and the overall temperature of the radio frequency operation object doesn't meet the requirements. Therefore, one perfusion channel that has been opened is randomly closed to reduce the perfusion volume, and return to the step of determining whether the ratio of the third temperature in the temperatures of the multiple sites acquired in real time is greater than the fourth ratio until all the perfusion channels are closed.

If the ratio of the third temperature is not greater than the fourth ratio, step S706 of determining whether the ratio of the fourth temperature in the temperatures of the multiple sites acquired in real time is greater than the fifth ratio is executed;
If the ratio of the fourth temperature is greater than the fifth ratio, step S707 of determining whether there is an unopened perfusion channel is executed;
If there is an unopened perfusion channel, step S708 of randomly opening one unopened perfusion channel is executed, and return to step S706 until all perfusion channels are opened;
If there is no unopened perfusion channel, step S709 of increasing the flow rate of the perfusion channels according to a preset second increment is executed, and return to step S706 until reaching the preset temperature-control flow rate limit;
If the ratio of the fourth temperature is not greater than the fifth ratio, return to step S704.

Specifically, if the ratio of the third temperature that is lower than the preset minimum temperature in the temperatures of the multiple sites acquired in real time is not greater than the fourth ratio, it means that the overall temperature of the radio frequency operation object is expected, and the current perfusion volume is conducive to the temperature rise of the radio frequency operation object. In order to avoid damage to the radio frequency operation object due to excessive temperature, it is determined whether the ratio of the fourth temperature in the temperatures of the multiple sites acquired in real time is greater than the fifth ratio. The fourth temperature is greater than the preset maximum temperature. The fourth ratio and the fifth ratio can be preset in the execution body of the radio frequency operation data control method herein as user defined.

On the one hand, if the ratio of the fourth temperature in the temperatures of the multiple sites acquired in real time is greater than the fifth ratio, it means that the overall temperature of the radio frequency operation object is too high, and there is a risk of damage and it is necessary to increase the perfusion volume to lower the temperature, and thus it is determined whether there is an unopened perfusion channel. If there is an unopened perfusion channel, randomly open one unopened perfusion channel, and return to the step of determining whether the ratio of the fourth temperature in the temperatures of the multiple sites acquired in real time is greater than the fifth ratio until all perfusion channels are opened aisle. If all the perfusion channels have been opened, increase the flow rate of the perfusion channels according to the preset second increment, and return to the step of determining whether the ratio of the fourth temperature in the temperatures of the multiple sites acquired in real time is greater than the fifth ratio until reaching the preset temperature-control flow rate limit. Furthermore, after reaching the preset temperature-control flow rate limit, if the ratio of the fourth temperature in the temperatures of the multiple sites acquired in real time is still greater than the fifth ratio, an early warning message is output.

On the other hand, if the ratio of the fourth temperature in the temperatures of the multiple sites acquired in real time is not greater than the fifth ratio, return to the step of determining whether the ratio of the third temperature in the temperatures of the multiple sites acquired in real time is greater than the fourth ratio.

According to the real-time changes of the temperature value, gradually increasing or decreasing the number of the perfusion channels to gradually increase or decrease the perfusion volume can improve the accuracy of perfusion control, reduce operational risks, and achieve better operation effect.

Step S710, when the flow rate of the syringe pump reaches the temperature-control limit, and if the temperature values of the multiple sites exceed the temperature protection range, controlling the output power of the radio frequency to make the temperature values of the multiple sites fall within the temperature protection range;
In the above step, the radio frequency operation object is controlled by adjusting the flow rate of the perfusion liquid. When the current flow rate reaches the temperature-control limit, the temperature will not be controlled by changing the flow rate. Instead, the temperature will be further controlled by controlling the output power of the radio frequency to adjust the temperature values of the multiple sites to be within the temperature protection range.

Step S711, detecting the impedance values of the multiple sites, and when the impedance values of the multiple sites exceed the preset impedance protection range, controlling the syringe pump to adjust the flow rates of part or all of the perfusion channels to make the impedance values of the multiple sites fall within the impedance protection range.

When the temperature is controlled to a normal value, that is, when it falls back within the temperature protection range, the impedance values of the multiple sites are detected. When the impedance values of the multiple sites exceed the preset impedance protection range, the syringe pump is controlled to adjust the flow rates of part or all of the perfusion channels to make the impedance values of the multiple sites fall within the impedance protection range. Adjusting the impedance values by controlling the syringe pump to adjust the flow rates of part or all of the perfusion channels can refer to the aforementioned description of adjusting the temperature values by controlling the syringe pump to adjust the flow rates of part or all of the perfusion channels.

The details not described here can refer to the relevant description in the embodiments shown in FIG. 4 to FIG. 6.

In the embodiment of the present disclosure, when the radio frequency operation task is triggered, the radio frequency operation catheter is firstly controlled to perform radio frequency operation, and after waiting for a preset time period, the syringe pump is controlled to open all the perfusion channels to perfuse the liquid through the opened perfusion channels into the radio frequency operation object at the initial flow rate, and the control mode for the radio frequency operation data is determined. In case of a dual control mode, according to the temperatures of multiple different sites of the radio frequency operation object acquired in real time by the plurality of temperature acquisition devices, the syringe pump is controlled to open or close part or all of the perfusion channels and / or to adjust the flow rates of part or all of the perfusion channels; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature changes of multiple different sites of the radio frequency operation object. Since the perfusion volume of the syringe pump is adjusted more purposefully and directionally according to the real-time changes of the temperature values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

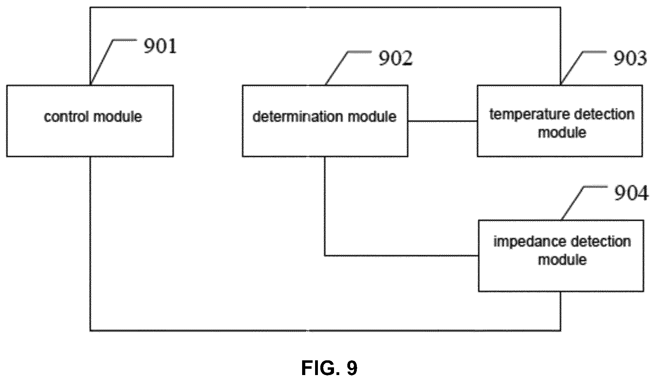

Referring to FIG. 9, a schematic diagram of a control apparatus for multi-channel perfusion of a syringe pump according to an embodiment of the present disclosure is shown. For illustration, only parts related to the embodiment of the present disclosure are shown. The device can be the syringe pump 20 shown in FIG. 1, the radio frequency host 10 or other computer terminals, or virtual modules operated in the above-mentioned devices. The device is configured to control the syringe pump with multiple perfusion channels, and specifically includes a control module 901, a determination module 902, a temperature detection module 903 and an impedance detection module 904.

The control module 901 is configured to, when the radio frequency operation task is triggered, control the syringe pump to open at least one perfusion channel, so as to perform a perfusion operation through the opened perfusion channel at a preset initial flow rate;
The determination module 902 is configured to determine the control mode for the radio frequency operation data;
The temperature detection module 902 is configured to detect the temperature values of multiple sites of the radio frequency operation object in real time if the control mode is a dual control mode;
The control module 901 is further configured to control the syringe pump to open or close part or all of the perfusion channels, and / or, control the syringe pump to adjust the flow rates of part or all of the perfusion channels according to the real-time changes of the temperature values of the multiple sites when the temperature values of the multiple sites exceed the preset temperature protection range;
The control module 901 is further configured to, when the flow rate of the syringe pump reaches the temperature-control limit and the temperature values of the multiple sites exceed the temperature protection range, control the output power of the radio frequency to make the temperature values of the multiple sites fall within the temperature protection range;
The impedance detection module 903 is configured to detect the impedance values of the multiple sites;
The control module 901 is further configured to, when the impedance values of the multiple sites exceed the preset impedance protection range, control the syringe pump to adjust the flow rates of part or all of the perfusion channels to make the impedance values of the multiple sites fall within the impedance protection range.

Further, the determination module 902 is further configured to obtain the user's setting on the data control mode; if the user has set the data control mode, then the control mode for the radio frequency operation data is determined according to the user's setting, and the data control mode is one of single, temperature control mode, single, impedance control mode or dual control mode; if the user has not set the data control mode, then the dual control mode is determined as the control mode for the radio frequency operation data.

Optionally, the control module 901 includes:
A first control sub-module configured to, when the radio frequency operation task is triggered, control the syringe pump to randomly open one perfusion channel, so as to perfuse liquid through the opened perfusion channel into the radio frequency operation object at the initial flow rate;
The first control sub-module is further configured to control the radio frequency operation catheter to perform radio frequency operation on the radio frequency operation object.

Optionally, the control module 901 further includes:
A second control sub-module configured for:
Determining whether there is a first temperature in the temperatures of the multiple sites acquired in real time, the first temperature being greater than a preset maximum temperature;
If there is the first temperature, it is determined whether the first perfusion channel for liquid perfusion to the first site is opened, and the first temperature is the temperature of the first site;
If the first perfusion channel is not opened, then the syringe pump is controlled to open the first perfusion channel, and return to the step of determining whether there is the first temperature in the temperatures of the multiple sites acquired in real time;
If the first perfusion channel has been opened, then the syringe pump is controlled to increase the flow rate of the first perfusion channel according to a preset first increment, and return to the step of determining whether there is the first temperature in the temperatures of the multiple sites acquired in real time steps until the flow rate of the first perfusion channel reaches the preset temperature-control flow rate limit.

Optionally, the second control sub-module is further configured for:
After determining whether there is the first temperature in the temperatures of the multiple sites, and if there is no first temperature, then determining whether the ratio of the first temperature acquisition device in the temperature acquisition devices is greater than the first ratio, and the temperature acquired by the first temperature acquisition device is less than the preset minimum temperature for a preset time period;
If the ratio is greater than the first ratio, the syringe pump is controlled to reduce the flow rate of the second perfusion channel according to the preset first decrement, and return to the step of determining whether the ratio of the first temperature acquisition device in the temperature acquisition devices is greater than the first ratio, until the flow rate of the second perfusion channel reaches the preset minimum flow rate, the second perfusion channel is configured to perfuse liquid into the second site, and the first temperature acquisition device is configured to detect the temperature of the second site;
If the ratio is not greater than the first ratio, return to the step of determining whether there is the first temperature in the temperatures of the multiple sites acquired in real time.

Optionally, the device further includes:
A setting module configured to set the respective preset maximum and minimum temperatures for the respective temperature acquisition devices;
The second control sub-module is further configured to determine the first perfusion channel and the second perfusion channel according to the respective preset maximum and minimum temperatures.

Optionally, the control module 901 further includes:
A third control sub-module configured for:
Determining whether the ratio of the second temperature in the temperatures of the multiple sites acquired in real time is greater than the second ratio, the second temperature being greater than the preset maximum temperature;
If the ratio of the second temperature is greater than the second ratio, then determining the perfusion increment according to a preset increment rule;
Determining the quantity of the perfusion channels to be opened according to the perfusion increment and the initial flow rate, and determining the third perfusion channel according to the quantity of the perfusion channels to be opened and the first determination rule;
Controlling the syringe pump to open the third perfusion channel, and returning to the step of determining whether the ratio of the second temperature in the temperatures of the multiple sites acquired in real time is greater than the second ratio, until all perfusion channels are opened; if the third perfusion channel has been opened, opening the perfusion channel adjacent to the third perfusion channel.

Optionally, the third control sub-module is further configured to control the syringe pump to increase the flow rate of all the perfusion channels to the preset temperature-control flow rate limit if the ratio of the second temperature is still greater than the second ratio after opening all the perfusion channels.

Optionally, the preset increment rule is to determine the perfusion increment according to the difference between the maximum temperature in the temperatures of the multiple sites and the preset maximum temperature, and the difference between the maximum temperature and the preset maximum temperature is directly proportional to the perfusion increment;

The first determination rule is to determine the third perfusion channel according to the distance from the second temperature acquisition device and the quantity of the perfusion channels to be opened, in order from proximal to distal, and the temperature acquired by the second temperature acquisition device is the maximum temperature.

Optionally, the third control sub-module is further configured for:
If the ratio of the second temperature is not greater than the second ratio, then determining whether the ratio of the third temperature acquisition device in the temperature acquisition devices is greater than the third ratio, the temperature acquired by the third temperature acquisition device being less than the preset minimum temperature;
If the ratio of the third temperature acquisition device is greater than the third ratio, then determining the perfusion decrement according to a preset decrement rule;
Determining the quantity of the perfusion channels to be closed according to the perfusion decrement and the initial flow rate, and determining the fourth perfusion channel according to the quantity of the perfusion channels to be closed and the second determination rule;
Controlling the syringe pump to close the fourth perfusion channel, and returning to the step of determining whether the ratio of the third temperature acquisition device in the temperature acquisition devices is greater than the third ratio until all perfusion channels are closed; if the fourth perfusion channel has been closed, then closing the perfusion channel adjacent to the fourth perfusion channel;
If the ratio of the third temperature acquisition device is not greater than the third ratio, returning to the step of determining whether the ratio of the second temperature in the temperatures of the multiple sites acquired in real time is greater than the second ratio.

Optionally, the preset decrement rule is to determine the perfusion decrement according to the difference between the lowest temperature in the temperatures of the multiple sites and the preset minimum temperature. The difference between the minimum temperature and the preset minimum temperature is directly proportional to the perfusion decrement.

The second determination rule is to determine the fourth perfusion channel according to the distance from the fourth temperature acquisition device in order from proximal to distal and the quantity of the perfusion channels to be closed. The temperature acquired by the fourth temperature acquisition device is the lowest.

Optionally, the control module 901 further includes:
A fourth control sub-module configured to control the radio frequency operation catheter to perform radio frequency operation when the radio frequency operation task is triggered;
The fourth control sub-module is further configured to control the syringe pump to open all the perfusion channels after waiting for a preset time period, so as to perfuse the liquid into the radio frequency operation object through the opened perfusion channels at the initial flow rate.

Optionally, the fourth control sub-module is further configured for:
Determining whether the ratio of the third temperature in the temperatures of the multiple sites acquired in real time is greater than the fourth ratio, and the third temperature being lower than the preset minimum temperature;
If the ratio of the third temperature is greater than the fourth ratio, randomly closing one perfusion channel that has been opened, and returning to the step of determining whether the ratio of the third temperature in the temperatures of the multiple sites acquired in real time is greater than the fourth ratio until all perfusion channels are closed;
If the ratio of the third temperature is not greater than the fourth ratio, determining whether the ratio of the fourth temperature in the temperatures of the multiple sites acquired in real time is greater than the fifth ratio, and the fourth temperature being greater than the preset maximum temperature;
If the ratio of the fourth temperature is greater than the fifth ratio, then determining whether there is an unopened perfusion channel;
If there is an unopened perfusion channel, randomly opening one unopened perfusion channel, and returning to the step of determining whether the ratio of the fourth temperature in the temperatures of the multiple sites acquired in real time is greater than the fifth ratio, until all the perfusion channels are opened;
If there is no unopened perfusion channel, increasing the flow rate of the perfusion channel according to the preset second increment, and returning to the step of determining whether the ratio of the fourth temperature in the temperatures of the multiple sites acquired in real time is greater than the ratio of the fifth ratio until reaching the preset temperature-control flow rate limit;
If the ratio of the fourth temperature is not greater than the fifth ratio, returning to the step of determining whether the ratio of the third temperature in the temperatures of the multiple sites acquired in real time is greater than the fourth ratio.

The specific process for realizing the functions of the above-mentioned modules can refer to the relevant description in the embodiments shown in FIG. 4 to FIG. 9, which will not be repeated here.

In the embodiment of the present disclosure, by using multiple syringe arrangements, when the radio frequency operation task is triggered, the syringe pump is controlled to open and perform the perfusion operation through at least one perfusion channel at the preset initial flow rate, and the control mode for the radio frequency operation data is determined. In case of a dual control mode, the temperature of multiple different sites of the radio frequency operation object is acquired in real time, and the syringe pump is controlled to open or close part or all of the perfusion channels, and / or to adjust the flow rates of part or all of the perfusion channels to control the temperature; and when the flow rate reaches the temperature-control limit, the output power of the radio frequency is adjusted to control the temperature. After the temperature control is over, the impedance of the radio frequency operation object is detected. If the impedance value exceeds the preset impedance protection range, the impedance value is adjusted by continuing to adjust the flow rate of the syringe pump, so as to realize the dual control of the temperature and impedance of the radio frequency operation object during the radio frequency operation, improving the effect of radio frequency operation and the safety of radio frequency operation, and to realize the intelligent and dynamic adjustment of multi-channel perfusion of the syringe pump based on the real-time temperature and impedance changes of multiple different sites of the radio frequency operation object. Since the perfusion volume of the syringe pump is adjusted more purposefully and directionally according to the real-time changes of the temperature and impedance values of different sites of the radio frequency operation object, operation delay and operation error caused by human judgment can be reduced, and the timeliness, accuracy and pertinence of liquid perfusion during the radio frequency operation can also be improved, thereby reducing the damage of radio frequency operation to the radio frequency operation object and improving the safety of radio frequency operation.

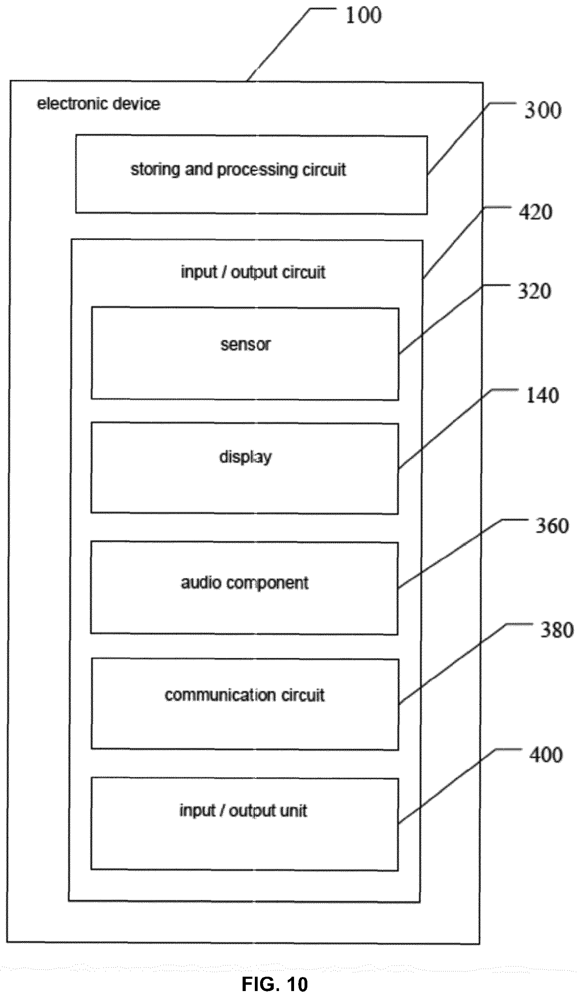

Referring to FIG. 10, a schematic structural diagram of hardware of an electronic apparatus according to an embodiment of the present disclosure is shown.

Exemplarily, the electronic apparatus may be any of various types of non-removable or removable or portable computer system devices performing wireless or wired communications. Specifically, the electronic apparatus may be a desktop computer, a server, a mobile phone or a smart phone (for example, a phone based on iPhone TM or Android TM); a portable game device (for example, Nintendo DS TM, PlayStation Portable TM, Gameboy Advance TM, iPhone TM), a laptop, a PDA, a portable Internet device, a music player, and a data storage device; other handheld device such as watch, earphones, pendant, headphone; or other wearable device such as electronic glasses, electronic cloth, electronic bracelet, electronic necklace, smart watch and head-mounted device ( HMD). In some cases, the electronic apparatus can perform various functions, for example, playing music, displaying video, storing images, and receiving and sending phone calls.

As shown in FIG. 10, the electronic apparatus 100 may include a control circuit, which may include a storing and processing circuit 300. The storing and processing circuit 300 may include a memory, such as hard disk drive memory, non-transitory or non-volatile memory (such as flash memory or other electronically programmable limited-erasable memory for forming solid-state drive, etc.), volatile memory (such as static or dynamic random access memory, etc.), and the like, which is not limited in this embodiment of the present disclosure. The processing circuit in the storing and processing circuit 300 may be configured to control the operation of the electronic apparatus 100. The processing circuit may be embodied based on one or more microprocessors, microcontrollers, digital signal processors, baseband processors, power management units, audio codec chips, application specific integrated circuits, display driver integrated circuits, and the like. The processor can be electrically coupled with a plurality of temperature acquisition devices (such as micro temperature sensors).

The storing and processing circuit 300 can be configured to run software in the electronic apparatus 100, such as Internet browsing applications, Voice over Internet Protocol (VOIP) telephone call applications, email applications, media player applications, operating system functions, etc. The software can be configured to perform control operations, such as image acquisition based on camera, ambient light measurement based on ambient light sensor, proximity sensor measurement based on proximity sensor, information display based on status indicators such as LEDs, touch event detection based on touch sensor, information display on a plurality of (e.g. layered) displayers, operations related to wireless communication, operations related to audio signal collection and generation, control operations related to data collection and process of button press event, and other functions in the electronic apparatus 100, which are not limited in the present embodiment of the present disclosure.

Further, the memory stores executable program codes, and the processor coupled to the memory is configured to invoke the executable program codes stored in the memory to execute the radio frequency operation data control method described in the embodiments referring to FIG. 4 to FIG. 7 above.

The executable program codes include various modules provided in the control apparatus for multi-channel perfusion of a syringe pump described in the above-mentioned embodiment shown in FIG. 9, such as the control module 901, determination module 902, temperature detection module 903 and impedance detection module 904. The respective functions of the control module 901, determination module 902, temperature detection module 903 and impedance detection module 904 can refer to the embodiment shown in FIG. 9, which will not be repeated here.

The electronic apparatus 100 may further include an input / output circuit 420. The input / output circuit 420 can be configured to enable the electronic apparatus 100 to realize data input and output, that is, allow the electronic apparatus 100 to receive data from external devices and also allow the electronic apparatus 100 to output data from the electronic apparatus 100 to external devices. The input / output circuit 420 may further include a sensor 320. The sensor 320 may be an ambient light sensor, a proximity sensor based on light and capacitance, a touch sensor (for example, a light and/ or capacitance based touch sensor which can be provided as a part of the touch panel or used independently), acceleration sensor, or other sensors, etc.

The input / output circuit 420 may further include one or more displays, such as display 140. The display 140 may include one or more of liquid crystal display, organic light emitting diode display, electronic ink display, plasma display, and displays using other display technologies. The display 140 may include a touch sensor array, that is, the display 140 may be a touch panel. The touch sensor may be a capacitive touch sensor formed from an array of transparent touch sensor electrodes such as indium tin oxide (ITO) electrodes, or may be a touch sensor formed using other touch technologies, such as acoustic touch, pressure sensitive touch, resistive touch, optical touch, etc., which are not limited in the present embodiment of the disclosure.

The electronic apparatus 100 may further include an audio component 360. The audio component 360 may be configured to provide audio input and output for the electronic apparatus 100. The audio component 360 in the electronic apparatus 100 may include speaker, microphone, buzzer, tone generator and other components for generating and detecting sounds.

A communication circuit 380 can be configured to provide the electronic apparatus 100 with the ability to communicate with external devices. The communication circuit 380 may include an analog and digital input / output interface circuit, and a wireless communication circuit based on radio frequency signals and / or optical signals. The wireless communication circuit in the communication circuit 380 may include a radio frequency transceiver circuit, a power amplifier circuit, a low noise amplifier, a switch, a filter, and an antenna. For example, the wireless communication circuit in the communication circuit 380 may include a circuit for supporting Near Field Communication (NFC) by transmitting and receiving near field coupled electromagnetic signals. For example, the communication circuit 380 may include a near field communication antenna and a near field communication transceiver. The communication circuit 380 may further include a cellular telephone transceiver and antennas, a wireless local area network transceiver circuit and antennas, and the like.

The electronic apparatus 100 may further include a battery, a power management circuit and other input / output unit 400. The input / output unit 400 may include a button, joystick, click wheel, scroll wheel, touch pad, keypad, keyboard, camera, light emitting diode and other status indicators, and the like.

The user can input instructions through the input / output circuit 420 to control the operation of the electronic apparatus 100, and can receive status information and other outputs from the electronic apparatus 100 through the output data of the input / output circuit 420.

Further, the embodiment of the present disclosure provides a computer-readable storage medium, which can be arranged in the electronic apparatus according to the above-mentioned embodiments, and the computer-readable storage medium can be the memory in the storing and processing circuit 300 described in the embodiment referring to FIG. 10. The computer-readable storage medium stores a computer program through which the radio frequency operation data control method according to the embodiments shown in FIG. 4 to FIG. 7 can be implemented when executed by the processor. Further, the computer storage medium may be a USB disk, a mobile hard disk, a Read-Only Memory (ROM), RAM, a magnetic disk or an optical disk, or other mediums that can store program codes.

In the several embodiments provided in this disclosure, it should be understood that the disclosed devices and methods may be implemented in other manners. For example, the devices according to the embodiments described above are only illustrative. For example, the division of the modules is only a division from the perspective of logical function, and in practice, other division manners can be used. For example, a plurality of modules or components can be combined or integrated into another system, or some features may be omitted or don't work. In another aspect, the mutual coupling or direct coupling or communication connection shown or discussed may be indirect coupling or communication connection between devices or modules via some interfaces, in electrical, mechanical or other forms.

The modules described as separate components may or may not be physically separated, and the components displayed as modules may or may not be physical modules, that is, they may be located in one place, or may be distributed on a plurality of network modules. Part or all of the modules can be used according to practical needs to achieve the purposes of the solutions of the embodiments.

In addition, the functional modules in each embodiment of the present disclosure may be integrated into one processing module, or may present separately physically, or two or more modules may be integrated into one module. The above-mentioned integrated modules can be implemented in the form of hardware or in the form of software modules.

If the integrated modules are implemented in the form of software modules and sold or used as independent products, they can be stored in a computer-readable storage medium. In view of this, the technical solution of the present disclosure or the part that contributes to the prior art or all or part of the technical solution can be embodied in the form of software which can be stored in a readable medium, including several instructions to enable a computer device (which may be a personal computer, server, or network device, etc.) to execute all or part of the steps of the methods described in the various embodiments of the present disclosure. The aforementioned readable storage medium includes various mediums capable of storing program codes, such as USB disk, mobile hard disk, ROM, RAM, magnetic disk or optical disk.

## Claims

1. An electronic apparatus, comprising:
a non-transitory memory and a processor;
wherein the non-transitory memory stores executable program codes;
the processor is electrically coupled with the non-transitory memory and a plurality of temperature acquisition devices and impedance acquisition devices (22); and
the processor is configured to invoke the executable program codes stored in the non-transitory memory to execute a radio frequency operation data control method, wherein
the radio frequency operation data control method comprises the steps of:
controlling (S401) a syringe pump (20) to open at least one perfusion channel when a radio frequency operation task is triggered, so as to perform perfusion operation through the opened perfusion channel at a preset initial flow rate;
determining (S402) a control mode for the radio frequency operation data, and when the control mode is a dual control mode, then:
detecting temperature values of multiple sites of a radio frequency operation object in real time;
controlling (S403) the syringe pump (20) to open or close part or all of the perfusion channels, and / or, controlling the syringe pump (20) to adjust flow rates of part or all of the perfusion channels according to real-time changes of the temperature values of the multiple sites when the temperature values of the multiple sites exceed a preset temperature protection range;
controlling (S404) an output power of the radio frequency to make the temperature values of the multiple sites fall within the temperature protection range when the flow rate of the syringe pump (20) reaches a temperature-control limit and the temperature values of the multiple sites exceed the temperature protection range; and
after a temperature control is over, detecting (S405) impedance values of the multiple sites, and when the impedance values of the multiple sites exceed a preset impedance protection range, controlling the syringe pump (20) to adjust the flow rates of part or all of the perfusion channels to make the impedance values of the multiple sites fall within the impedance protection range,
wherein the step of determining the control mode for the radio frequency operation data comprises:
acquiring user's setting on the control mode;
when the user has set the control mode, then determining the control mode for the radio frequency operation data according to the user's setting, the control mode being one of single, temperature control mode, single, impedance control mode or dual control mode; and
when the user has not set the control mode, then determining the dual control mode as the control mode for the radio frequency operation data.

2. The electronic apparatus according to claim 1, wherein the step of controlling (S401) the syringe pump (20) to open at least one perfusion channel when the radio frequency operation task is triggered, so as to perform perfusion operation through the opened perfusion channel at the preset initial flow rate comprises:
controlling (S501/S601) the syringe pump (20) to randomly open one perfusion channel when the radio frequency operation task is triggered, so as to perfuse liquid into the radio frequency operation object through the opened perfusion channel at the initial flow rate,
and wherein after the step of controlling the syringe pump (20) to randomly open one perfusion channel when the radio frequency operation task is triggered, the method further comprises:
controlling a radio frequency operation catheter to perform radio frequency operation on the radio frequency operation object.

3. The electronic apparatus according to claim 1 or 2, wherein the step of controlling the syringe pump (20) to open or close part or all of the perfusion channels, and / or, controlling the syringe pump (20) to adjust flow rates of part or all of the perfusion channels according to real-time changes of the temperature values of the multiple sites comprises:
determining (S503) whether there is a first temperature in temperatures of the multiple sites acquired in real time, the first temperature being greater than a preset maximum temperature;
when there is the first temperature, determining (S504) whether a first perfusion channel is opened, the first perfusion channel being configured to perfuse liquid into a first site, and the first temperature being the temperature of the first site;
when the first perfusion channel is not opened, controlling (S505) the syringe pump (20) to open the first perfusion channel, and returning to the step of determining (S503) whether there is the first temperature in the temperatures of the multiple sites acquired in real time;
when the first perfusion channel has been opened, controlling (S506) the syringe pump (20) to increase the flow rate of the first perfusion channel according to a preset first increment, and returning to the step of determining (S503) whether there is the first temperature in the temperatures of the multiple sites acquired in real time, until the flow rate of the first perfusion channel reaches the preset temperature-control flow rate limit;
when there is no first temperature, determining (S507) whether a ratio of a first temperature acquisition device in temperature acquisition devices is greater than a first ratio, the temperature acquired by the first temperature acquisition device being less than a preset minimum temperature for a preset time period;
when the ratio of the first temperature acquisition device is greater than the first ratio, controlling (S508) the syringe pump (20) to reduce the flow rate of a second perfusion channel according to a preset first decrement, and returning to the step of determining (S507) whether the ratio of the first temperature acquisition device in the temperature acquisition devices is greater than the first ratio, until the flow rate of the second perfusion channel reaches a preset minimum flow rate, wherein the second perfusion channel is configured to perfuse liquid to a second site, and the first temperature acquisition device is configured to detect the temperature of the second site; and
when the ratio of the first temperature acquisition device is not greater than the first ratio, returning to the step of determining whether there is the first temperature in the temperatures of the multiple sites acquired in real time.

4. The electronic apparatus according to claim 3, wherein the method further comprises setting respective preset maximum and minimum temperatures for each of the temperature acquisition devices, and determining the first perfusion channel and the second perfusion channel according to the respective preset maximum and minimum temperatures.

5. The electronic apparatus according to claim 1 or 2, wherein the step of controlling the syringe pump (20) to open or close part or all of the perfusion channels, and / or, controlling the syringe pump (20) to adjust flow rates of part or all of the perfusion channels according to real-time changes of the temperature values of the multiple sites comprises:
determining (S603) whether a ratio of a second temperature in temperatures of the multiple sites acquired in real time is greater than a second ratio, the second temperature being greater than a preset maximum temperature;
when the ratio of the second temperature is greater than the second ratio, then determining (S604) a perfusion increment according to a preset increment rule;
determining (S605) a quantity of the perfusion channels to be opened according to the perfusion increment and the initial flow rate, and determining a third perfusion channel according to the quantity of the perfusion channels to be opened and a first determination rule;
controlling (S606) the syringe pump (20) to open the third perfusion channel, and returning to the step of determining (S603) whether the ratio of the second temperature in the temperatures of the multiple sites acquired in real time is greater than the second ratio, until all perfusion channels are opened, and when the third perfusion channel has been opened, then opening the perfusion channel adjacent to the third perfusion channel; and
after opening all perfusion channels, when the ratio of the second temperature is still greater than the second ratio, controlling the syringe pump (20) to increase the flow rates of all perfusion channels to the preset temperature-control flow rate limit.

6. The electronic apparatus according to claim 5, wherein the preset increment rule is to determine the perfusion increment according to a difference between the maximum temperature in the temperatures of the multiple sites and the preset maximum temperature, and wherein the difference between the maximum temperature and the preset maximum temperature is directly proportional to the perfusion increment; and the first determination rule is to determine the third perfusion channel according to a distance from a second temperature acquisition device and the quantity of the perfusion channels to be opened in order from proximal to distal, wherein the second temperature acquisition device is configured to acquire the maximum temperature.

7. The electronic apparatus according to claim 1, wherein the step of controlling the syringe pump (20) to open at least one perfusion channel when the radio frequency operation task is triggered, so as to perform perfusion operation through the opened perfusion channel at the preset initial flow rate comprises:
controlling (S701) a radio frequency operation catheter to perform radio frequency operation when the radio frequency operation task is triggered; and
after waiting for a preset time period, controlling (S702) the syringe pump (20) to open all the perfusion channels, so as to perfuse liquid into the radio frequency operation object through the opened perfusion channels at the initial flow rate.

8. The electronic apparatus according to claim 7, wherein the step of controlling the syringe pump (20) to open or close part or all of the perfusion channels, and / or, controlling the syringe pump (20) to adjust flow rates of part or all of the perfusion channels according to real-time changes of the temperature values of the multiple sites comprises:
determining (S704) whether a ratio of a third temperature in temperatures of the multiple sites acquired in real time is greater than a fourth ratio, the third temperature being lower than a preset minimum temperature;
when the ratio of the third temperature is greater than the fourth ratio, randomly closing (S705) one perfusion channel that has been opened, and returning to the step of determining (S704) whether the ratio of the third temperature in the temperatures of the multiple sites acquired in real time is greater than the fourth ratio until all perfusion channels are closed;
when the ratio of the third temperature is not greater than the fourth ratio, determining (S706) whether a ratio of a fourth temperature in the temperatures of the multiple sites acquired in real time is greater than a fifth ratio, the fourth temperature being greater than a preset maximum temperature;
when the ratio of the fourth temperature is greater than the fifth ratio, determining (S707) whether there is an unopened perfusion channel;
when there is an unopened perfusion channel, randomly opening (S708) one unopened perfusion channel, and returning to the step of determining (S706) whether the ratio of the fourth temperature in the temperatures of the multiple sites acquired in real time is greater than the fifth ratio, until all perfusion channels are opened;
when there is no unopened perfusion channel, increasing (S709) the flow rate of the perfusion channels according to a preset second increment, and returning to the step of determining (S706) whether the ratio of the fourth temperature in the temperatures of the multiple sites acquired in real time is greater than the fifth ratio until reaching the preset temperature-control flow rate limit; and
when the ratio of the fourth temperature is not greater than the fifth ratio, returning to the step of determining (S704) whether the ratio of the third temperature in the temperatures of the multiple sites acquired in real time is greater than the fourth ratio.

9. A syringe pump (20), comprising a controller (21), a plurality of temperature and impedance acquisition devices (22), and multiple syringe arrangements (23); wherein each syringe arrangement (23) comprises a syringe (231), an extension tube (232), a push rod (233) and a driving device (234), and wherein one end of the extension tube (232) is connected to the syringe (231), and the other end is provided with at least one of the temperature and impedance acquisition devices (22), and each syringe arrangement (23) defines a perfusion channel; and
the controller (21) is electrically coupled with the plurality of temperature and impedance acquisition devices (22), electrically connected with the multiple syringe arrangements (23), and configured to execute the steps of a radio frequency operation data control method, wherein the radio frequency operation data control method comprises the steps of:
controlling (S401) the syringe pump (20) to open at least one perfusion channel when a radio frequency operation task is triggered, so as to perform perfusion operation through the opened perfusion channel at a preset initial flow rate;
determining (S402) a control mode for the radio frequency operation data, and when the control mode is a dual control mode, then:
detecting temperature values of multiple sites of a radio frequency operation object in real time;
controlling (S403) the syringe pump (20) to open or close part or all of the perfusion channels, and / or, controlling the syringe pump (20) to adjust flow rates of part or all of the perfusion channels according to real-time changes of the temperature values of the multiple sites when the temperature values of the multiple sites exceed a preset temperature protection range;
controlling (S404) an output power of the radio frequency to make the temperature values of the multiple sites fall within the temperature protection range when the flow rate of the syringe pump (20) reaches a temperature-control limit and the temperature values of the multiple sites exceed the temperature protection range; and
after a temperature control is over, detecting (S405) impedance values of the multiple sites, and when the impedance values of the multiple sites exceed a preset impedance protection range, controlling the syringe pump (20) to adjust the flow rates of part or all of the perfusion channels to make the impedance values of the multiple sites fall within the impedance protection range,
wherein the step of determining the control mode for the radio frequency operation data comprises:
acquiring user's setting on the control mode;
when the user has set the control mode, then determining the control mode for the radio frequency operation data according to the user's setting, the control mode being one of single, temperature control mode, single, impedance control mode or dual control mode; and
when the user has not set the control mode, then determining the dual control mode as the control mode for the radio frequency operation data.

10. The syringe pump (20) according to claim 9, wherein the step of controlling (S401) the syringe pump (20) to open at least one perfusion channel when the radio frequency operation task is triggered, so as to perform perfusion operation through the opened perfusion channel at the preset initial flow rate comprises:
controlling (S501/S601) the syringe pump (20) to randomly open one perfusion channel when the radio frequency operation task is triggered, so as to perfuse liquid into the radio frequency operation object through the opened perfusion channel at the initial flow rate,
and wherein after the step of controlling the syringe pump (20) to randomly open one perfusion channel when the radio frequency operation task is triggered, the method further comprises:
controlling a radio frequency operation catheter to perform radio frequency operation on the radio frequency operation object.

11. The syringe pump (20) according to claim 9 or 10, wherein the step of controlling the syringe pump (20) to open or close part or all of the perfusion channels, and / or, controlling the syringe pump (20) to adjust flow rates of part or all of the perfusion channels according to real-time changes of the temperature values of the multiple sites comprises:
determining (S503) whether there is a first temperature in temperatures of the multiple sites acquired in real time, the first temperature being greater than a preset maximum temperature;
when there is the first temperature, determining (S504) whether a first perfusion channel is opened, the first perfusion channel being configured to perfuse liquid into a first site, and the first temperature being the temperature of the first site;
when the first perfusion channel is not opened, controlling (S505) the syringe pump (20) to open the first perfusion channel, and returning to the step of determining (S503) whether there is the first temperature in the temperatures of the multiple sites acquired in real time;
when the first perfusion channel has been opened, controlling (S506) the syringe pump (20) to increase the flow rate of the first perfusion channel according to a preset first increment, and returning to the step of determining (S503) whether there is the first temperature in the temperatures of the multiple sites acquired in real time, until the flow rate of the first perfusion channel reaches the preset temperature-control flow rate limit;
when there is no first temperature, determining (S507) whether a ratio of a first temperature acquisition device in temperature acquisition devices is greater than a first ratio, the temperature acquired by the first temperature acquisition device being less than a preset minimum temperature for a preset time period;
when the ratio of the first temperature acquisition device is greater than the first ratio, controlling (S508) the syringe pump (20) to reduce the flow rate of a second perfusion channel according to a preset first decrement, and returning to the step of determining (S507) whether the ratio of the first temperature acquisition device in the temperature acquisition devices is greater than the first ratio, until the flow rate of the second perfusion channel reaches a preset minimum flow rate, wherein the second perfusion channel is configured to perfuse liquid to a second site, and the first temperature acquisition device is configured to detect the temperature of the second site; and
when the ratio of the first temperature acquisition device is not greater than the first ratio, returning to the step of determining whether there is the first temperature in the temperatures of the multiple sites acquired in real time.

12. The syringe pump (20) according to claim 11, wherein the method further comprises setting respective preset maximum and minimum temperatures for each of the temperature acquisition devices, and determining the first perfusion channel and the second perfusion channel according to the respective preset maximum and minimum temperatures.

13. The syringe pump (20) according to claim 9 or 10, wherein the step of controlling the syringe pump (20) to open or close part or all of the perfusion channels, and / or, controlling the syringe pump (20) to adjust flow rates of part or all of the perfusion channels according to real-time changes of the temperature values of the multiple sites comprises:
determining (S603) whether a ratio of a second temperature in temperatures of the multiple sites acquired in real time is greater than a second ratio, the second temperature being greater than a preset maximum temperature;
when the ratio of the second temperature is greater than the second ratio, then determining (S604) a perfusion increment according to a preset increment rule;
determining (S605) a quantity of the perfusion channels to be opened according to the perfusion increment and the initial flow rate, and determining a third perfusion channel according to the quantity of the perfusion channels to be opened and a first determination rule;
controlling (S606) the syringe pump (20) to open the third perfusion channel, and returning to the step of determining (S603) whether the ratio of the second temperature in the temperatures of the multiple sites acquired in real time is greater than the second ratio, until all perfusion channels are opened, and when the third perfusion channel has been opened, then opening the perfusion channel adjacent to the third perfusion channel; and
after opening all perfusion channels, when the ratio of the second temperature is still greater than the second ratio, controlling the syringe pump (20) to increase the flow rates of all perfusion channels to the preset temperature-control flow rate limit.

14. The syringe pump (20) according to claim 13, wherein the preset increment rule is to determine the perfusion increment according to a difference between the maximum temperature in the temperatures of the multiple sites and the preset maximum temperature, and wherein the difference between the maximum temperature and the preset maximum temperature is directly proportional to the perfusion increment; and the first determination rule is to determine the third perfusion channel according to a distance from a second temperature acquisition device and the quantity of the perfusion channels to be opened in order from proximal to distal, wherein the second temperature acquisition device is configured to acquire the maximum temperature.

15. The syringe pump (20) according to claim 9, wherein the step of controlling the syringe pump (20) to open at least one perfusion channel when the radio frequency operation task is triggered, so as to perform perfusion operation through the opened perfusion channel at the preset initial flow rate comprises:
controlling (S701) a radio frequency operation catheter to perform radio frequency operation when the radio frequency operation task is triggered; and
after waiting for a preset time period, controlling (S702) the syringe pump (20) to open all the perfusion channels, so as to perfuse liquid into the radio frequency operation object through the opened perfusion channels at the initial flow rate.

## Patentansprüche

1. Elektronische Vorrichtung, umfassend:
einen nicht-flüchtigen Speicher und einen Prozessor;
worin der nicht-flüchtige Speicher ausführbare Programmcodes speichert;
der Prozessor mit dem nicht-flüchtigen Speicher und einer Vielzahl von Temperaturerfassungsvorrichtungen und Impedanzerfassungsvorrichtungen (22) elektrisch gekoppelt ist; und
der Prozessor dazu konfiguriert ist, die ausführbaren Programmcodes aufzurufen, die im nicht-flüchtigen Speicher gespeichert sind, um ein Hochfrequenzoperationsdaten-Steuerungsverfahren auszuführen, worin das Hochfrequenzoperationsdaten-Steuerungsverfahren die Schritte umfasst:
Steuern (S401) einer Spritzenpumpe (20), um zumindest einen Perfusionskanal zu öffnen, wenn eine Hochfrequenzoperationsaufgabe ausgelöst wird, so dass ein Perfusionsvorgang durch den geöffneten Perfusionskanal bei einer vorgegebenen Anfangsdurchflussmenge durchgeführt wird;
Bestimmen (S402) eines Steuerungsmodus für die Hochfrequenzoperationsdaten, und, wenn der Steuerungsmodus ein Doppelsteuerungsmodus ist, dann:
Detektieren von Temperaturwerten von mehreren Stellen eines Hochfrequenzoperationsobjektes in Echtzeit;
Steuern (S403) der Spritzenpumpe (20), um einen Teil oder alle Perfusionskanäle zu öffnen oder zu schließen, und / oder Steuern der Spritzenpumpe (20), um die Durchflussmengen eines Teils oder aller Perfusionskanäle gemäß Echtzeitänderungen der Temperaturwerte der mehreren Stellen einzustellen, wenn die Temperaturwerte der mehreren Stellen einen vorgegebenen Temperaturschutzbereich überschreiten;
Steuern (S404) einer Ausgangsleistung der Hochfrequenz, um zu bewirken, dass die Temperaturwerte der mehreren Stellen in den Temperaturschutzbereich fallen, wenn die Durchflussmenge der Spritzenpumpe (20) eine Temperatursteuerungsgrenze erreicht und die Temperaturwerte der mehreren Stellen den Temperaturschutzbereich überschreiten; und
nachdem eine Temperatursteuerung zu Ende ist, Detektieren (S405) von Impedanzwerten der mehreren Stellen, und, wenn die Impedanzwerte der mehreren Stellen einen vorgegebenen Impedanzschutzbereich überschreiten, Steuern der Spritzenpumpe (20), um die Durchflussmengen eines Teils oder aller Perfusionskanäle einzustellen, damit die Impedanzwerte der mehreren Stellen in den Impedanzschutzbereich fallen,
worin der Schritt des Bestimmens des Steuerungsmodus für die Hochfrequenzoperationsdaten umfasst:
Erfassen der Benutzereinstellung am Steuerungsmodus;
wenn der Benutzer den Steuerungsmodus festgelegt hat, dann Bestimmen des Steuerungsmodus für die Hochfrequenzoperationsdaten gemäß der Benutzereinstellung, wobei der Steuerungsmodus einer von Einzel-Temperatursteuerungsmodus, Einzel-Impedanzsteuerungsmodus oder Doppelsteuerungsmodus ist; und
wenn der Benutzer den Steuerungsmodus nicht festgelegt hat, dann Bestimmen des Doppelsteuerungsmodus als Steuerungsmodus für die Hochfrequenzoperationsdaten.

2. Elektronische Vorrichtung nach Anspruch 1, worin der Schritt des Steuerns (S401) der Spritzenpumpe (20), um zumindest einen Perfusionskanal zu öffnen, wenn die Hochfrequenzoperationsaufgabe ausgelöst wird, so dass ein Perfusionsvorgang durch den geöffneten Perfusionskanal bei der vorgegebenen Anfangsdurchflussmenge durchgeführt wird, umfasst:
Steuern (S501/S601) der Spritzenpumpe (20), um einen Perfusionskanal zufällig zu öffnen, wenn die Hochfrequenzoperationsaufgabe ausgelöst wird, so dass Flüssigkeit in das Hochfrequenzoperationsobjekt durch den geöffneten Perfusionskanal bei der Anfangsdurchflussmenge perfundiert wird,
und worin, nach dem Schritt des Steuerns der Spritzenpumpe (20), um einen Perfusionskanal zufällig zu öffnen, wenn die Hochfrequenzoperationsaufgabe ausgelöst wird, das Verfahren ferner umfasst:
Steuern eines Hochfrequenzoperationskatheters, um Hochfrequenzoperation am Hochfrequenzoperationsobjekt durchzuführen.

3. Elektronische Vorrichtung nach Anspruch 1 oder 2, worin der Schritt des Steuerns der Spritzenpumpe (20), um einen Teil oder alle Perfusionskanäle zu öffnen oder zu schließen, und / oder des Steuerns der Spritzenpumpe (20), um die Durchflussmengen eines Teils oder aller Perfusionskanäle gemäß Echtzeitänderungen der Temperaturwerte der mehreren Stellen einzustellen, umfasst:
Bestimmen (S503), ob es eine erste Temperatur in den Temperaturen der mehreren Stellen, die in Echtzeit erfasst werden, gibt, wobei die erste Temperatur höher ist als eine vorgegebene Höchsttemperatur;
wenn es die erste Temperatur gibt, Bestimmen (S504), ob ein erster Perfusionskanal geöffnet ist, wobei der erste Perfusionskanal dazu eingerichtet ist, Flüssigkeit in eine erste Stelle zu perfundieren, und die erste Temperatur die Temperatur der ersten Stelle ist;
wenn der erste Perfusionskanal nicht geöffnet ist, Steuern (S505) der Spritzenpumpe (20), um den ersten Perfusionskanal zu öffnen, und Zurückkehren zum Schritt des Bestimmens (S503), ob es die erste Temperatur in den Temperaturen der mehreren Stellen, die in Echtzeit erfasst werden, gibt;
wenn der erste Perfusionskanal geöffnet worden ist, Steuern (S506) der Spritzenpumpe (20), um die Durchflussmenge des ersten Perfusionskanals gemäß einer vorgegebenen ersten Zunahme zu erhöhen, und Zurückkehren zum Schritt des Bestimmens (S503), ob es die erste Temperatur in den Temperaturen der mehreren Stellen, die in Echtzeit erfasst werden, gibt, bis die Durchflussmenge des ersten Perfusionskanals die vorgegebene Temperatur-Steuerungsdurchflussmengengrenze erreicht; wenn es keine erste Temperatur gibt, Bestimmen (S507), ob ein Verhältnis einer ersten Temperaturerfassungsvorrichtung in den Temperaturerfassungsvorrichtungen größer ist als ein erstes Verhältnis, wobei die Temperatur, die von der ersten Temperaturerfassungsvorrichtung erfasst wird, kleiner ist als eine vorgegebene Mindesttemperatur für einen vorgegebenen Zeitraum;
wenn das Verhältnis der ersten Temperaturerfassungsvorrichtung größer ist als das erste Verhältnis, Steuern (S508) der Spritzenpumpe (20), um die Durchflussmenge eines zweiten Perfusionskanals gemäß einer vorgegebenen ersten Abnahme zu reduzieren, und Zurückkehren zum Schritt des Bestimmens (S507), ob das Verhältnis der ersten Temperaturerfassungsvorrichtung in den Temperaturerfassungsvorrichtungen größer ist als das erste Verhältnis, bis die Durchflussmenge des zweiten Perfusionskanals eine vorgegebene Mindestdurchflussmenge erreicht, worin der zweite Perfusionskanal dafür ausgelegt ist, Flüssigkeit zu einer zweiten Stelle zu perfundieren, und die erste Temperaturerfassungsvorrichtung dafür ausgelegt ist, die Temperatur der zweiten Stelle zu detektieren; und
wenn das Verhältnis der ersten Temperaturerfassungsvorrichtung nicht größer ist als das erste Verhältnis, Zurückkehren zum Schritt des Bestimmens, ob es die erste Temperatur in den Temperaturen der mehreren Stellen, die in Echtzeit erfasst werden, gibt.

4. Elektronische Vorrichtung nach Anspruch 3, worin das Verfahren ferner das Festlegen von jeweiligen vorgegebenen Höchst- und Mindesttemperaturen für jede der Temperaturerfassungsvorrichtungen, und Bestimmen des ersten Perfusionskanals und des zweiten Perfusionskanals gemäß den jeweiligen vorgegebenen Höchst- und Mindesttemperaturen umfasst.

5. Elektronische Vorrichtung nach Anspruch 1 oder 2, worin der Schritt des Steuerns der Spritzenpumpe (20), um einen Teil oder alle Perfusionskanäle zu öffnen oder zu schließen, und / oder Steuerns der Spritzenpumpe (20), um die Durchflussmengen eines Teils oder aller Perfusionskanäle gemäß Echtzeitänderungen der Temperaturwerte der mehreren Stellen einzustellen, umfasst:
Bestimmen (S603), ob ein Verhältnis einer zweiten Temperatur in Temperaturen der mehreren Stellen, die in Echtzeit erfasst werden, größer ist als ein zweites Verhältnis, wobei die zweite Temperatur größer ist als eine vorgegebene Höchsttemperatur;
wenn das Verhältnis der zweiten Temperatur größer ist als das zweite Verhältnis, dann Bestimmen (S604) einer Perfusionszunahme gemäß einer vorgegebenen Zunahmeregel;
Bestimmen (S605) einer Menge der Perfusionskanäle, die geöffnet werden sollen, gemäß der Perfusionszunahme und der Anfangsdurchflussmenge, und Bestimmen eines dritten Perfusionskanals gemäß der Menge der Perfusionskanäle, die geöffnet werden sollen, und einer ersten Bestimmungsregel;
Steuern (S606) der Spritzenpumpe (20), um den dritten Perfusionskanal zu öffnen, und Zurückkehren zum Schritt des Bestimmens (S603), ob das Verhältnis der zweiten Temperatur in den Temperaturen der mehreren Stellen, die in Echtzeit erfasst werden, größer ist als das zweite Verhältnis, bis alle Perfusionskanäle geöffnet sind, und wenn der dritte Perfusionskanal geöffnet worden ist, dann Öffnen des Perfusionskanals, der an den dritten Perfusionskanal angrenzend ist; und
nach dem Öffnen aller Perfusionskanäle, wenn das Verhältnis der zweiten Temperatur noch größer ist als das zweite Verhältnis, Steuern der Spritzenpumpe (20), um die Durchflussmengen aller Perfusionskanäle auf die vorgegebene Temperatur-Steuerungsdurchflussmengengrenze zu erhöhen.

6. Elektronische Vorrichtung nach Anspruch 5, worin die vorgegebene Zunahmeregel es ist, die Perfusionszunahme gemäß einem Unterschied zwischen der Höchsttemperatur in den Temperaturen der mehreren Stellen und der vorgegebenen Höchsttemperatur zu bestimmen, und worin der Unterschied zwischen der Höchsttemperatur und der vorgegebenen Höchsttemperatur zur Perfusionszunahme direkt proportional ist; und
die erste Bestimmungsregel es ist, den dritten Perfusionskanal gemäß einem Abstand von einer zweiten Temperaturerfassungsvorrichtung und der Menge der Perfusionskanäle, die geöffnet werden sollen, in Reihenfolge von proximal zu distal zu bestimmen, worin die zweite Temperaturerfassungsvorrichtung dafür ausgelegt ist, die Höchsttemperatur zu erfassen.

7. Elektronische Vorrichtung nach Anspruch 1, worin der Schritt des Steuerns der Spritzenpumpe (20), um zumindest einen Perfusionskanal zu öffnen, wenn die Hochfrequenzoperationsaufgabe ausgelöst wird, so dass ein Perfusionsvorgang durch den geöffneten Perfusionskanal bei der vorgegebenen Anfangsdurchflussmenge durchgeführt wird, umfasst:
Steuern (S701) eines Hochfrequenzoperationskatheters, um Hochfrequenzoperation durchzuführen, wenn die Hochfrequenzoperationsaufgabe ausgelöst wird; und
nach dem Warten für einen vorgegebenen Zeitraum, Steuern (S702) der Spritzenpumpe (20), um alle Perfusionskanäle zu öffnen, um Flüssigkeit in das Hochfrequenzoperationsobjekt durch die geöffneten Perfusionskanäle bei der Anfangsdurchflussmenge zu perfundieren.

8. Elektronische Vorrichtung nach Anspruch 7, worin der Schritt des Steuerns der Spritzenpumpe (20), um einen Teil oder alle Perfusionskanäle zu öffnen oder zu schließen, und / oder Steuerns der Spritzenpumpe (20), um die Durchflussmengen eines Teils oder aller Perfusionskanäle gemäß Echtzeitänderungen der Temperaturwerte der mehreren Stellen einzustellen, umfasst:
Bestimmen (S704), ob ein Verhältnis einer dritten Temperatur in Temperaturen der mehreren Stellen, die in Echtzeit erfasst werden, größer ist als ein viertes Verhältnis, wobei die dritte Temperatur niedriger ist als eine vorgegebene Mindesttemperatur;
wenn das Verhältnis der dritten Temperatur größer ist als das vierte Verhältnis, zufälliges Schließen (S705) eines Perfusionskanals, der geöffnet worden ist, und Zurückkehren zum Schritt des Bestimmens (S704), ob das Verhältnis der dritten Temperatur in den Temperaturen der mehreren Stellen, die in Echtzeit erfasst werden, größer ist als das vierte Verhältnis, bis alle Perfusionskanäle geschlossen sind;
wenn das Verhältnis der dritten Temperatur nicht größer ist als das vierte Verhältnis, Bestimmen (S706), ob ein Verhältnis einer vierten Temperatur in den Temperaturen der mehreren Stellen, die in Echtzeit erfasst werden, größer ist als ein fünftes Verhältnis, wobei die vierte Temperatur höher ist als eine vorgegebene Höchsttemperatur;
wenn das Verhältnis der vierten Temperatur größer ist als das fünfte Verhältnis, Bestimmen (S707), ob es einen ungeöffneten Perfusionskanal gibt;
wenn es einen ungeöffneten Perfusionskanal gibt, zufälliges Öffnen (S708) eines ungeöffneten Perfusionskanals, und Zurückkehren zum Schritt des Bestimmens (S706), ob das Verhältnis der vierten Temperatur in den Temperaturen der mehreren Stellen, die in Echtzeit erfasst werden, größer ist als das fünfte Verhältnis, bis alle Perfusionskanäle geöffnet sind;
wenn es keinen ungeöffneten Perfusionskanal gibt, Erhöhen (S709) der Durchflussmenge der Perfusionskanäle gemäß einer vorgegebenen zweiten Zunahme, und Zurückkehren zum Schritt des Bestimmens (S706), ob das Verhältnis der vierten Temperatur in den Temperaturen der mehreren Stellen, die in Echtzeit erfasst werden, größer ist als das fünfte Verhältnis, bis die vorgegebene Temperatur-Steuerungsdurchflussmengengrenze erreicht wird; und
wenn das Verhältnis der vierten Temperatur nicht größer ist als das fünfte Verhältnis, Zurückkehren zum Schritt des Bestimmens (S704), ob das Verhältnis der dritten Temperatur in den Temperaturen der mehreren Stellen, die in Echtzeit erfasst werden, größer ist als das vierte Verhältnis.

9. Spritzenpumpe (20), umfassend ein Steuergerät (21), eine Vielzahl von Temperatur- und Impedanzerfassungsvorrichtungen (22) und mehrere Spritzenanordnungen (23); worin
jede Spritzenanordnung (23) eine Spritze (231), ein Verlängerungsrohr (232), eine Schubstange (233) und eine Antriebsvorrichtung (234) umfasst, und worin ein Ende des Verlängerungsrohres (232) mit der Spritze (231) verbunden ist, und das andere Ende mit zumindest einer der Temperatur- und Impedanzerfassungsvorrichtungen (22) versehen ist, und jede Spritzenanordnung (23) einen Perfusionskanal definiert; und
das Steuergerät (21) mit der Vielzahl von Temperatur- und Impedanzerfassungsvorrichtungen (22) elektrisch gekoppelt ist, mit den mehreren Spritzenanordnungen (23) elektrisch verbunden ist, und dazu konfiguriert ist, die Schritte eines Hochfrequenzoperationsdaten-Steuerungsverfahrens auszuführen, worin das Hochfrequenzoperationsdaten-Steuerungsverfahren die Schritte umfasst:
Steuern (S401) der Spritzenpumpe (20), um zumindest einen Perfusionskanal zu öffnen, wenn eine Hochfrequenzoperationsaufgabe ausgelöst wird, so dass ein Perfusionsvorgang durch den geöffneten Perfusionskanal bei einer vorgegebenen Anfangsdurchflussmenge durchgeführt wird;
Bestimmen (S402) eines Steuerungsmodus für die Hochfrequenzoperationsdaten, und, wenn der Steuerungsmodus ein Doppelsteuerungsmodus ist, dann:
Detektieren von Temperaturwerten von mehreren Stellen eines Hochfrequenzoperationsobjektes in Echtzeit;
Steuern (S403) der Spritzenpumpe (20), um einen Teil oder alle Perfusionskanäle zu öffnen oder zu schließen, und / oder Steuern der Spritzenpumpe (20), um die Durchflussmengen eines Teils oder aller Perfusionskanäle gemäß Echtzeitänderungen der Temperaturwerte der mehreren Stellen einzustellen, wenn die Temperaturwerte der mehreren Stellen einen vorgegebenen Temperaturschutzbereich überschreiten;
Steuern (S404) einer Ausgangsleistung der Hochfrequenz, um zu bewirken, dass die Temperaturwerte der mehreren Stellen in den Temperaturschutzbereich fallen, wenn die Durchflussmenge der Spritzenpumpe (20) eine Temperatursteuerungsgrenze erreicht und die Temperaturwerte der mehreren Stellen den Temperaturschutzbereich überschreiten; und
nachdem eine Temperatursteuerung zu Ende ist, Detektieren (S405) von Impedanzwerten der mehreren Stellen, und, wenn die Impedanzwerte der mehreren Stellen einen vorgegebenen Impedanzschutzbereich überschreiten, Steuern der Spritzenpumpe (20), um die Durchflussmengen eines Teils oder aller Perfusionskanäle einzustellen, damit die Impedanzwerte der mehreren Stellen in den Impedanzschutzbereich fallen,
worin der Schritt des Bestimmens des Steuerungsmodus für die Hochfrequenzoperationsdaten umfasst:
Erfassen der Benutzereinstellung am Steuerungsmodus;
wenn der Benutzer den Steuerungsmodus festgelegt hat, dann Bestimmen des Steuerungsmodus für die Hochfrequenzoperationsdaten gemäß der Benutzereinstellung, wobei der Steuerungsmodus einer von Einzel-Temperatursteuerungsmodus, Einzel-Impedanzsteuerungsmodus oder Doppelsteuerungsmodus ist; und
wenn der Benutzer den Steuerungsmodus nicht festgelegt hat, dann Bestimmen des Doppelsteuerungsmodus als Steuerungsmodus für die Hochfrequenzoperationsdaten.

10. Spritzenpumpe (20) nach Anspruch 9, worin der Schritt des Steuerns (S401) der Spritzenpumpe (20), um zumindest einen Perfusionskanal zu öffnen, wenn die Hochfrequenzoperationsaufgabe ausgelöst wird, so dass ein Perfusionsvorgang durch den geöffneten Perfusionskanal bei der vorgegebenen Anfangsdurchflussmenge durchgeführt wird, umfasst:
Steuern (S501/S601) der Spritzenpumpe (20), um einen Perfusionskanal zufällig zu öffnen, wenn die Hochfrequenzoperationsaufgabe ausgelöst wird, so dass Flüssigkeit in das Hochfrequenzoperationsobjekt durch den geöffneten Perfusionskanal bei der Anfangsdurchflussmenge perfundiert wird,
und worin, nach dem Schritt des Steuerns der Spritzenpumpe (20), um einen Perfusionskanal zufällig zu öffnen, wenn die Hochfrequenzoperationsaufgabe ausgelöst wird, das Verfahren ferner umfasst:
Steuern eines Hochfrequenzoperationskatheters, um Hochfrequenzoperation am Hochfrequenzoperationsobjekt durchzuführen.

11. Spritzenpumpe (20) nach Anspruch 9 oder 10, worin der Schritt des Steuerns der Spritzenpumpe (20), um einen Teil oder alle Perfusionskanäle zu öffnen oder zu schließen, und / oder des Steuerns der Spritzenpumpe (20), um die Durchflussmengen eines Teils oder aller Perfusionskanäle gemäß Echtzeitänderungen der Temperaturwerte der mehreren Stellen einzustellen, umfasst:
Bestimmen (S503), ob es eine erste Temperatur in den Temperaturen der mehreren Stellen, die in Echtzeit erfasst werden, gibt, wobei die erste Temperatur höher ist als eine vorgegebene Höchsttemperatur;
wenn es die erste Temperatur gibt, Bestimmen (S504), ob ein erster Perfusionskanal geöffnet ist, wobei der erste Perfusionskanal dazu eingerichtet ist, Flüssigkeit in eine erste Stelle zu perfundieren, und die erste Temperatur die Temperatur der ersten Stelle ist;
wenn der erste Perfusionskanal nicht geöffnet ist, Steuern (S505) der Spritzenpumpe (20), um den ersten Perfusionskanal zu öffnen, und Zurückkehren zum Schritt des Bestimmens (S503), ob es die erste Temperatur in den Temperaturen der mehreren Stellen, die in Echtzeit erfasst werden, gibt;
wenn der erste Perfusionskanal geöffnet worden ist, Steuern (S506) der Spritzenpumpe (20), um die Durchflussmenge des ersten Perfusionskanals gemäß einer vorgegebenen ersten Zunahme zu erhöhen, und Zurückkehren zum Schritt des Bestimmens (S503), ob es die erste Temperatur in den Temperaturen der mehreren Stellen, die in Echtzeit erfasst werden, gibt, bis die Durchflussmenge des ersten Perfusionskanals die vorgegebene Temperatur-Steuerungsdurchflussmengengrenze erreicht; wenn es keine erste Temperatur gibt, Bestimmen (S507), ob ein Verhältnis einer ersten Temperaturerfassungsvorrichtung in den Temperaturerfassungsvorrichtungen größer ist als ein erstes Verhältnis, wobei die Temperatur, die von der ersten Temperaturerfassungsvorrichtung erfasst wird, kleiner ist als eine vorgegebene Mindesttemperatur für einen vorgegebenen Zeitraum;
wenn das Verhältnis der ersten Temperaturerfassungsvorrichtung größer ist als das erste Verhältnis, Steuern (S508) der Spritzenpumpe (20), um die Durchflussmenge eines zweiten Perfusionskanals gemäß einer vorgegebenen ersten Abnahme zu reduzieren, und Zurückkehren zum Schritt des Bestimmens (S507), ob das Verhältnis der ersten Temperaturerfassungsvorrichtung in den Temperaturerfassungsvorrichtungen größer ist als das erste Verhältnis, bis die Durchflussmenge des zweiten Perfusionskanals eine vorgegebene Mindestdurchflussmenge erreicht, worin der zweite Perfusionskanal dafür ausgelegt ist, Flüssigkeit zu einer zweiten Stelle zu perfundieren, und die erste Temperaturerfassungsvorrichtung dafür ausgelegt ist, die Temperatur der zweiten Stelle zu detektieren; und
wenn das Verhältnis der ersten Temperaturerfassungsvorrichtung nicht größer ist als das erste Verhältnis, Zurückkehren zum Schritt des Bestimmens, ob es die erste Temperatur in den Temperaturen der mehreren Stellen, die in Echtzeit erfasst werden, gibt.

12. Spritzenpumpe (20) nach Anspruch 11, worin das Verfahren ferner das Festlegen von jeweiligen vorgegebenen Höchst- und Mindesttemperaturen für jede der Temperaturerfassungsvorrichtungen, und Bestimmen des ersten Perfusionskanals und des zweiten Perfusionskanals gemäß den jeweiligen vorgegebenen Höchst- und Mindesttemperaturen umfasst.

13. Spritzenpumpe (20) nach Anspruch 9 oder 10, worin der Schritt des Steuerns der Spritzenpumpe (20), um einen Teil oder alle Perfusionskanäle zu öffnen oder zu schließen, und / oder Steuerns der Spritzenpumpe (20), um die Durchflussmengen eines Teils oder aller Perfusionskanäle gemäß Echtzeitänderungen der Temperaturwerte der mehreren Stellen einzustellen, umfasst:
Bestimmen (S603), ob ein Verhältnis einer zweiten Temperatur in Temperaturen der mehreren Stellen, die in Echtzeit erfasst werden, größer ist als ein zweites Verhältnis, wobei die zweite Temperatur größer ist als eine vorgegebene Höchsttemperatur;
wenn das Verhältnis der zweiten Temperatur größer ist als das zweite Verhältnis, dann Bestimmen (S604) einer Perfusionszunahme gemäß einer vorgegebenen Zunahmeregel;
Bestimmen (S605) einer Menge der Perfusionskanäle, die geöffnet werden sollen, gemäß der Perfusionszunahme und der Anfangsdurchflussmenge, und Bestimmen eines dritten Perfusionskanals gemäß der Menge der Perfusionskanäle, die geöffnet werden sollen, und einer ersten Bestimmungsregel;
Steuern (S606) der Spritzenpumpe (20), um den dritten Perfusionskanal zu öffnen, und Zurückkehren zum Schritt des Bestimmens (S603), ob das Verhältnis der zweiten Temperatur in den Temperaturen der mehreren Stellen, die in Echtzeit erfasst werden, größer ist als das zweite Verhältnis, bis alle Perfusionskanäle geöffnet sind, und wenn der dritte Perfusionskanal geöffnet worden ist, dann Öffnen des Perfusionskanals, der an den dritten Perfusionskanal angrenzend ist; und
nach dem Öffnen aller Perfusionskanäle, wenn das Verhältnis der zweiten Temperatur noch größer ist als das zweite Verhältnis, Steuern der Spritzenpumpe (20), um die Durchflussmengen aller Perfusionskanäle auf die vorgegebene Temperatur-Steuerungsdurchflussmengengrenze zu erhöhen.

14. Spritzenpumpe (20) nach Anspruch 13, worin die vorgegebene Zunahmeregel es ist, die Perfusionszunahme gemäß einem Unterschied zwischen der Höchsttemperatur in den Temperaturen der mehreren Stellen und der vorgegebenen Höchsttemperatur zu bestimmen, und worin der Unterschied zwischen der Höchsttemperatur und der vorgegebenen Höchsttemperatur zur Perfusionszunahme direkt proportional ist; und
die erste Bestimmungsregel es ist, den dritten Perfusionskanal gemäß einem Abstand von einer zweiten Temperaturerfassungsvorrichtung und der Menge der Perfusionskanäle, die geöffnet werden sollen, in Reihenfolge von proximal zu distal zu bestimmen, worin die zweite Temperaturerfassungsvorrichtung dafür ausgelegt ist, die Höchsttemperatur zu erfassen.

15. Spritzenpumpe (20) nach Anspruch 9, worin der Schritt des Steuerns der Spritzenpumpe (20), um zumindest einen Perfusionskanal zu öffnen, wenn die Hochfrequenzoperationsaufgabe ausgelöst wird, so dass ein Perfusionsvorgang durch den geöffneten Perfusionskanal bei der vorgegebenen Anfangsdurchflussmenge durchgeführt wird, umfasst:
Steuern (S701) eines Hochfrequenzoperationskatheters, um Hochfrequenzoperation durchzuführen, wenn die Hochfrequenzoperationsaufgabe ausgelöst wird; und
nach dem Warten für einen vorgegebenen Zeitraum, Steuern (S702) der Spritzenpumpe (20), um alle Perfusionskanäle zu öffnen, um Flüssigkeit in das Hochfrequenzoperationsobjekt durch die geöffneten Perfusionskanäle bei der Anfangsdurchflussmenge zu perfundieren.

## Revendications

1. Appareil électronique, comprenant :
une mémoire non transitoire et un processeur ;
dans lequel la mémoire non transitoire stocke des codes de programme exécutables ;
le processeur est électriquement couplé à la mémoire non transitoire et à une pluralité de dispositifs d'acquisition de température et de dispositifs d'acquisition d'impédance (22) ; et
le processeur est configuré pour invoquer les codes de programme exécutables stockés dans la mémoire non transitoire pour exécuter un procédé de commande de données de fonctionnement en radiofréquence, dans lequel le procédé de commande de données de fonctionnement en radiofréquence comprend les étapes suivantes :
commander (S401) une pompe à seringue (20) pour ouvrir au moins un canal de perfusion lorsqu'une tâche de fonctionnement en radiofréquence est déclenchée, de manière à effectuer une opération de perfusion à travers le canal de perfusion ouvert à un débit initial prédéfini ;
déterminer (S402) un mode de commande pour les données de fonctionnement en radiofréquence, et lorsque le mode de commande est un mode de commande double, alors :
détecter les valeurs de température de plusieurs sites d'un objet de fonctionnement en radiofréquence en temps réel ;
commander (S403) la pompe à seringue (20) pour ouvrir ou fermer une partie ou la totalité des canaux de perfusion, et / ou, commander la pompe à seringue (20) pour ajuster les débits d'une partie ou de la totalité des canaux de perfusion selon les changements en temps réel des valeurs de température des sites multiples lorsque les valeurs de température des sites multiples dépassent une plage de protection de température prédéfinie ;
commander (S404) une puissance de sortie de la radiofréquence pour faire en sorte que les valeurs de température des sites multiples tombent dans la plage de protection contre la température lorsque le débit de la pompe à seringue (20) atteint une limite de commande de la température et que les valeurs de température des sites multiples dépassent la plage de protection contre la température ; et
après la fin d'une commande de température, détecter (S405) les valeurs d'impédance des sites multiples, et lorsque les valeurs d'impédance des sites multiples dépassent une plage de protection d'impédance prédéfinie, commander la pompe à seringue (20) pour ajuster les débits d'une partie ou de la totalité des canaux de perfusion afin que les valeurs d'impédance des sites multiples tombent dans la plage de protection d'impédance,
dans lequel l'étape de détermination du mode de commande pour les données de fonctionnement en radiofréquence comprend :
l'acquisition du réglage de l'utilisateur sur le mode de commande ;
lorsque l'utilisateur a défini le mode de commande, la détermination ensuite du mode de commande pour les données de fonctionnement en radiofréquence en fonction du réglage de l'utilisateur, le mode de commande étant l'un des modes suivants : mode de commande de température simple, mode de commande d'impédance simple ou mode de commande double ; et
lorsque l'utilisateur n'a pas défini le mode de commande, la détermination ensuite du mode de commande double en tant que mode de commande pour les données de fonctionnement en radiofréquence.

2. Appareil électronique selon la revendication 1, dans lequel l'étape de commande (S401) de la pompe à seringue (20) pour ouvrir au moins un canal de perfusion lorsque la tâche de fonctionnement en radiofréquence est déclenchée, de manière à effectuer une opération de perfusion à travers le canal de perfusion ouvert au débit initial prédéfini comprend :
la commande (S501/S601) de la pompe à seringue (20) pour ouvrir de manière aléatoire un canal de perfusion lorsque la tâche de fonctionnement en radiofréquence est déclenchée, de manière à perfuser du liquide dans l'objet de fonctionnement en radiofréquence à travers le canal de perfusion ouvert au débit initial,
et dans lequel, après l'étape de commande de la pompe à seringue (20) pour ouvrir de manière aléatoire un canal de perfusion lorsque la tâche de fonctionnement en radiofréquence est déclenchée, le procédé comprend en outre :
la commande d'un cathéter de fonctionnement en radiofréquence pour effectuer un fonctionnement en radiofréquence sur l'objet de fonctionnement en radiofréquence.

3. Appareil électronique selon la revendication 1 ou 2, dans lequel l'étape de commande de la pompe à seringue (20) pour ouvrir ou fermer une partie ou la totalité des canaux de perfusion, et / ou de commande de la pompe à seringue (20) pour ajuster les débits d'une partie ou de la totalité des canaux de perfusion en fonction des changements en temps réel des valeurs de température des sites multiples comprend :
la détermination (S503) s'il existe une première température dans les températures des multiples sites acquis en temps réel, la première température étant supérieure à une température maximale prédéfinie ;
lorsqu'il y a la première température, la détermination (S504) si un premier canal de perfusion est ouvert, le premier canal de perfusion étant configuré pour perfuser du liquide dans un premier site, et la première température étant la température du premier site ;
lorsque le premier canal de perfusion n'est pas ouvert, la commande (S505) de la pompe à seringue (20) pour ouvrir le premier canal de perfusion, et le retour à l'étape de détermination (S503) s'il y a la première température dans les températures des sites multiples acquis en temps réel ;
lorsque le premier canal de perfusion a été ouvert, la commande (S506) de la pompe à seringue (20) pour augmenter le débit du premier canal de perfusion en fonction d'un premier incrément prédéfini, et le retour à l'étape de détermination (S503) s'il y a la première température dans les températures des sites multiples acquis en temps réel, jusqu'à ce que le débit du premier canal de perfusion atteigne la limite de débit de commande de température prédéfinie ;
lorsqu'il n'y a pas de première température, la détermination (S507) si un rapport d'un premier dispositif d'acquisition de température dans des dispositifs d'acquisition de température est supérieur à un premier rapport, la température acquise par le premier dispositif d'acquisition de température étant inférieure à une température minimale prédéfinie pendant une période de temps prédéfinie ;
lorsque le rapport du premier dispositif d'acquisition de température est supérieur au premier rapport, la commande (S508) de la pompe à seringue (20) pour réduire le débit d'un deuxième canal de perfusion en fonction d'un premier décrément prédéfini, et le retour à l'étape de détermination (S507) si le rapport du premier dispositif d'acquisition de température dans les dispositifs d'acquisition de température est supérieur au premier rapport, jusqu'à ce que le débit du deuxième canal de perfusion atteigne un débit minimum prédéfini, dans lequel le deuxième canal de perfusion est configuré pour perfuser du liquide vers un second site, et le premier dispositif d'acquisition de température est configuré pour détecter la température du second site ; et
lorsque le rapport du premier dispositif d'acquisition de température n'est pas supérieur au premier rapport, le retour à l'étape de détermination s'il y a la première température dans les températures des sites multiples acquis en temps réel.

4. Appareil électronique selon la revendication 3, dans lequel le procédé comprend en outre le réglage des températures maximale et minimale prédéfinies respectives pour chacun des dispositifs d'acquisition de température, et la détermination du premier canal de perfusion et du deuxième canal de perfusion en fonction des températures maximale et minimale prédéfinies respectives.

5. Appareil électronique selon la revendication 1 ou 2, dans lequel l'étape de commande de la pompe à seringue (20) pour ouvrir ou fermer une partie ou la totalité des canaux de perfusion, et / ou de commande de la pompe à seringue (20) pour ajuster les débits d'une partie ou de la totalité des canaux de perfusion en fonction des changements en temps réel des valeurs de température des sites multiples comprend :
la détermination (S603) si un rapport d'une deuxième température dans les températures des sites multiples acquis en temps réel est supérieur à un deuxième rapport, la deuxième température étant supérieure à une température maximale prédéfinie ;
lorsque le rapport de la deuxième température est supérieur au deuxième rapport, la détermination (S604) ensuite d'un incrément de perfusion selon une règle d'incrément prédéfinie ;
la détermination (S605) d'une quantité des canaux de perfusion à ouvrir en fonction de l'incrément de perfusion et du débit initial, et la détermination d'un troisième canal de perfusion en fonction de la quantité des canaux de perfusion à ouvrir et d'une première règle de détermination ;
la commande (S606) de la pompe à seringue (20) pour ouvrir le troisième canal de perfusion, et le retour à l'étape de détermination (S603) si le rapport de la seconde température dans les températures des sites multiples acquis en temps réel est supérieur au deuxième rapport, jusqu'à ce que tous les canaux de perfusion soient ouverts, et lorsque le troisième canal de perfusion a été ouvert, puis ouvrir le canal de perfusion adjacent au troisième canal de perfusion ; et
après avoir ouvert tous les canaux de perfusion, lorsque le rapport de la deuxième température est encore supérieur au deuxième rapport, la commande de la pompe à seringue (20) pour augmenter les débits de tous les canaux de perfusion à la limite de débit de commande de température prédéfinie.

6. Appareil électronique selon la revendication 5, dans lequel la règle d'incrémentation prédéfinie est de déterminer l'incrément de perfusion selon une différence entre la température maximale dans les températures des sites multiples et la température maximale prédéfinie, et dans lequel la différence entre la température maximale et la température maximale prédéfinie est directement proportionnelle à l'incrément de perfusion ; et la première règle de détermination consiste à déterminer le troisième canal de perfusion en fonction d'une distance à partir d'un second dispositif d'acquisition de température et de la quantité des canaux de perfusion à ouvrir dans l'ordre du proximal au distal, dans lequel le second dispositif d'acquisition de température est configuré pour acquérir la température maximale.

7. Appareil électronique selon la revendication 1, dans lequel l'étape de commande de la pompe à seringue (20) pour ouvrir au moins un canal de perfusion lorsque la tâche de fonctionnement en radiofréquence est déclenchée, de manière à effectuer une opération de perfusion à travers le canal de perfusion ouvert au débit initial prédéfini comprend :
la commande (S701) d'un cathéter de fonctionnement en radiofréquence pour effectuer un fonctionnement en radiofréquence lorsque la tâche de fonctionnement en radiofréquence est déclenchée ; et
après avoir attendu une période de temps prédéfinie, la commande (S702) de la pompe à seringue (20) pour ouvrir tous les canaux de perfusion, de manière à perfuser du liquide dans l'objet de fonctionnement en radiofréquence à travers les canaux de perfusion ouverts au débit initial.

8. Appareil électronique selon la revendication 7, dans lequel l'étape de commande de la pompe à seringue (20) pour ouvrir ou fermer une partie ou la totalité des canaux de perfusion, et / ou de commande de la pompe à seringue (20) pour ajuster les débits d'une partie ou de la totalité des canaux de perfusion en fonction des changements en temps réel des valeurs de température des sites multiples comprend :
la détermination (S704) si un rapport d'une troisième température dans les températures des sites multiples acquis en temps réel est supérieur à un quatrième rapport, la troisième température étant inférieure à une température minimale prédéfinie ;
lorsque le rapport de la troisième température est supérieur au quatrième rapport, la fermeture aléatoire (S705) d'un canal de perfusion qui a été ouvert, et le retour à l'étape de détermination (S704) si le rapport de la troisième température dans les températures des sites multiples acquis en temps réel est supérieur au quatrième rapport jusqu'à ce que tous les canaux de perfusion soient fermés ;
lorsque le rapport de la troisième température n'est pas supérieur au quatrième rapport, la détermination (S706) si un rapport d'une quatrième température dans les températures des sites multiples acquis en temps réel est supérieur à un cinquième rapport, la quatrième température étant supérieure à une température maximale prédéfinie ;
lorsque le rapport de la quatrième température est supérieur au cinquième rapport, la détermination (S707) s'il existe un canal de perfusion non ouvert ;
lorsqu'il y a un canal de perfusion non ouvert, l'ouverture aléatoire (S708) d'un canal de perfusion non ouvert, et le retour à l'étape de détermination (S706) si le rapport de la quatrième température dans les températures des sites multiples acquis en temps réel est supérieur au cinquième rapport, jusqu'à ce que tous les canaux de perfusion soient ouverts ;
lorsqu'il n'y a pas de canal de perfusion non ouvert, l'augmentation (S709) du débit des canaux de perfusion en fonction d'un second incrément prédéfini, et le retour à l'étape de détermination (S706) si le rapport de la quatrième température dans les températures des sites multiples acquis en temps réel est supérieur au cinquième rapport jusqu'à atteindre la limite de débit de commande de la température prédéfinie ; et
lorsque le rapport de la quatrième température n'est pas supérieur au cinquième rapport, le retour à l'étape de détermination (S704) si le rapport de la troisième température dans les températures des sites multiples acquis en temps réel est supérieur au quatrième rapport.

9. Pompe à seringue (20), comprenant un dispositif de commande (21), une pluralité de dispositifs d'acquisition de température et d'impédance (22) et de multiples agencements de seringue (23) ; dans laquelle
chaque agencement de seringue (23) comprend une seringue (231), un tube d'extension (232), une tige de poussée (233) et un dispositif d'entraînement (234), et dans laquelle une extrémité du tube d'extension (232) est reliée à la seringue (231), et l'autre extrémité est pourvue d'au moins l'un des dispositifs d'acquisition de température et d'impédance (22), et chaque agencement de seringue (23) définit un canal de perfusion ; et
le dispositif de commande (21) est couplé électriquement à la pluralité de dispositifs d'acquisition de température et d'impédance (22), connecté électriquement aux multiples agencements de seringues (23), et configuré pour exécuter les étapes d'un procédé de commande de données de fonctionnement en radiofréquence, dans laquelle le procédé de commande de données de fonctionnement en radiofréquence comprend les étapes suivantes :
commander (S401) la pompe à seringue (20) pour ouvrir au moins un canal de perfusion lorsqu'une tâche de fonctionnement en radiofréquence est déclenchée, de manière à effectuer une opération de perfusion à travers le canal de perfusion ouvert à un débit initial prédéfini ;
déterminer (S402) un mode de commande pour les données de fonctionnement en radiofréquence, et lorsque le mode de commande est un mode de commande double, alors :
détecter les valeurs de température de plusieurs sites d'un objet de fonctionnement en radiofréquence en temps réel ;
commander (S403) la pompe à seringue (20) pour ouvrir ou fermer une partie ou la totalité des canaux de perfusion, et / ou, commander la pompe à seringue (20) pour ajuster les débits d'une partie ou de la totalité des canaux de perfusion selon les changements en temps réel des valeurs de température des sites multiples lorsque les valeurs de température des sites multiples dépassent une plage de protection de température prédéfinie ;
commander (S404) une puissance de sortie de la radiofréquence pour faire en sorte que les valeurs de température des sites multiples tombent dans la plage de protection contre la température lorsque le débit de la pompe à seringue (20) atteint une limite de commande de la température et que les valeurs de température des sites multiples dépassent la plage de protection contre la température ; et
après la fin d'une commande de température, détecter (S405) les valeurs d'impédance des sites multiples, et lorsque les valeurs d'impédance des sites multiples dépassent une plage de protection d'impédance prédéfinie, commander la pompe à seringue (20) pour ajuster les débits d'une partie ou de la totalité des canaux de perfusion afin que les valeurs d'impédance des sites multiples tombent dans la plage de protection d'impédance,
dans lequel l'étape de détermination du mode de commande pour les données de fonctionnement en radiofréquence comprend :
l'acquisition du réglage de l'utilisateur sur le mode de commande ;
lorsque l'utilisateur a défini le mode de commande, la détermination ensuite du mode de commande pour les données de fonctionnement en radiofréquence en fonction du réglage de l'utilisateur, le mode de commande étant l'un des modes suivants : mode de commande de température simple, mode de commande d'impédance simple ou mode de commande double ; et
lorsque l'utilisateur n'a pas défini le mode de commande, la détermination ensuite du mode de commande double en tant que mode de commande pour les données de fonctionnement en radiofréquence.

10. Pompe à seringue (20) selon la revendication 9, dans laquelle l'étape de commande (S401) de la pompe à seringue (20) pour ouvrir au moins un canal de perfusion lorsque la tâche de fonctionnement en radiofréquence est déclenchée, de manière à effectuer une opération de perfusion à travers le canal de perfusion ouvert au débit initial prédéfini comprend :
la commande (S501/S601) de la pompe à seringue (20) pour ouvrir de manière aléatoire un canal de perfusion lorsque la tâche de fonctionnement en radiofréquence est déclenchée, de manière à perfuser du liquide dans l'objet de fonctionnement en radiofréquence à travers le canal de perfusion ouvert au débit initial,
et dans lequel, après l'étape de commande de la pompe à seringue (20) pour ouvrir de manière aléatoire un canal de perfusion lorsque la tâche de fonctionnement en radiofréquence est déclenchée, le procédé comprend en outre :
la commande d'un cathéter de fonctionnement en radiofréquence pour effectuer un fonctionnement en radiofréquence sur l'objet de fonctionnement en radiofréquence.

11. Pompe à seringue (20) selon la revendication 9 ou 10, dans laquelle l'étape de commande de la pompe à seringue (20) pour ouvrir ou fermer une partie ou la totalité des canaux de perfusion, et / ou de commande de la pompe à seringue (20) pour ajuster les débits d'une partie ou de la totalité des canaux de perfusion en fonction des changements en temps réel des valeurs de température des sites multiples comprend :
la détermination (S503) s'il existe une première température dans les températures des multiples sites acquis en temps réel, la première température étant supérieure à une température maximale prédéfinie ;
lorsqu'il y a la première température, la détermination (S504) si un premier canal de perfusion est ouvert, le premier canal de perfusion étant configuré pour perfuser du liquide dans un premier site, et la première température étant la température du premier site ;
lorsque le premier canal de perfusion n'est pas ouvert, la commande (S505) de la pompe à seringue (20) pour ouvrir le premier canal de perfusion, et le retour à l'étape de détermination (S503) s'il y a la première température dans les températures des sites multiples acquis en temps réel ;
lorsque le premier canal de perfusion a été ouvert, la commande (S506) de la pompe à seringue (20) pour augmenter le débit du premier canal de perfusion en fonction d'un premier incrément prédéfini, et le retour à l'étape de détermination (S503) s'il y a la première température dans les températures des multiples sites acquis en temps réel, jusqu'à ce que le débit du premier canal de perfusion atteigne la limite de débit de commande de température prédéfinie ;
lorsqu'il n'y a pas de première température, la détermination (S507) si un rapport d'un premier dispositif d'acquisition de température dans des dispositifs d'acquisition de température est supérieur à un premier rapport, la température acquise par le premier dispositif d'acquisition de température étant inférieure à une température minimale prédéfinie pendant une période de temps prédéfinie ;
lorsque le rapport du premier dispositif d'acquisition de température est supérieur au premier rapport, la commande (S508) de la pompe à seringue (20) pour réduire le débit d'un deuxième canal de perfusion en fonction d'un premier décrément prédéfini, et le retour à l'étape de détermination (S507) si le rapport du premier dispositif d'acquisition de température dans les dispositifs d'acquisition de température est supérieur au premier rapport, jusqu'à ce que le débit du deuxième canal de perfusion atteigne un débit minimum prédéfini, dans lequel le deuxième canal de perfusion est configuré pour perfuser du liquide vers un second site, et le premier dispositif d'acquisition de température est configuré pour détecter la température du second site ; et
lorsque le rapport du premier dispositif d'acquisition de température n'est pas supérieur au premier rapport, le retour à l'étape de détermination s'il y a la première température dans les températures des sites multiples acquis en temps réel.

12. Pompe à seringue (20) selon la revendication 11, dans laquelle le procédé comprend en outre le réglage des températures maximale et minimale prédéfinies respectives pour chacun des dispositifs d'acquisition de température, et la détermination du premier canal de perfusion et du deuxième canal de perfusion en fonction des températures maximale et minimale prédéfinies respectives.

13. Pompe à seringue (20) selon la revendication 9 ou 10, dans laquelle l'étape de commande de la pompe à seringue (20) pour ouvrir ou fermer une partie ou la totalité des canaux de perfusion, et / ou de commande de la pompe à seringue (20) pour ajuster les débits d'une partie ou de la totalité des canaux de perfusion en fonction des changements en temps réel des valeurs de température des sites multiples comprend :
la détermination (S603) si un rapport d'une deuxième température dans les températures des sites multiples acquis en temps réel est supérieur à un deuxième rapport, la deuxième température étant supérieure à une température maximale prédéfinie ;
lorsque le rapport de la deuxième température est supérieur au deuxième rapport, la détermination (S604) ensuite d'un incrément de perfusion selon une règle d'incrément prédéfinie ;
la détermination (S605) d'une quantité des canaux de perfusion à ouvrir en fonction de l'incrément de perfusion et du débit initial, et la détermination d'un troisième canal de perfusion en fonction de la quantité des canaux de perfusion à ouvrir et d'une première règle de détermination ;
la commande (S606) de la pompe à seringue (20) pour ouvrir le troisième canal de perfusion, et le retour à l'étape de détermination (S603) si le rapport de la seconde température dans les températures des sites multiples acquis en temps réel est supérieur au deuxième rapport, jusqu'à ce que tous les canaux de perfusion soient ouverts, et lorsque le troisième canal de perfusion a été ouvert, puis ouvrir le canal de perfusion adjacent au troisième canal de perfusion ; et
après avoir ouvert tous les canaux de perfusion, lorsque le rapport de la deuxième température est encore supérieur au deuxième rapport, la commande de la pompe à seringue (20) pour augmenter les débits de tous les canaux de perfusion à la limite de débit de commande de température prédéfinie.

14. Pompe à seringue (20) selon la revendication 13, dans laquelle la règle d'incrémentation prédéfinie est de déterminer l'incrément de perfusion selon une différence entre la température maximale dans les températures des sites multiples et la température maximale prédéfinie, et dans lequel la différence entre la température maximale et la température maximale prédéfinie est directement proportionnelle à l'incrément de perfusion ; et la première règle de détermination consiste à déterminer le troisième canal de perfusion en fonction d'une distance à partir d'un second dispositif d'acquisition de température et de la quantité des canaux de perfusion à ouvrir dans l'ordre du proximal au distal, dans lequel le second dispositif d'acquisition de température est configuré pour acquérir la température maximale.

15. Pompe à seringue (20) selon la revendication 9, dans laquelle l'étape de commande de la pompe à seringue (20) pour ouvrir au moins un canal de perfusion lorsque la tâche de fonctionnement en radiofréquence est déclenchée, de manière à effectuer une opération de perfusion à travers le canal de perfusion ouvert au débit initial prédéfini comprend :
la commande (S701) d'un cathéter de fonctionnement en radiofréquence pour effectuer un fonctionnement en radiofréquence lorsque la tâche de fonctionnement en radiofréquence est déclenchée ; et
après avoir attendu une période de temps prédéfinie, la commande (S702) de la pompe à seringue (20) pour ouvrir tous les canaux de perfusion, de manière à perfuser du liquide dans l'objet de fonctionnement en radiofréquence à travers les canaux de perfusion ouverts au débit initial.
